Europäisches Patentamt

European Patent Office

Office européen des brevets

(19)

(11) **EP 1 333 286 A1**

(12) **EUROPEAN PATENT APPLICATION**
published in accordance with Art. 158(3) EPC

(43) Date of publication:
06.08.2003 Bulletin 2003/32

(51) Int Cl.⁷: **G01N 35/00**, G01N 37/00,
G01N 33/48, C12M 1/00,
C12Q 1/68

(21) Application number: 01965681.8

(22) Date of filing: 17.09.2001

(86) International application number:
PCT/JP01/08079

(87) International publication number:
WO 02/025289 (28.03.2002 Gazette 2002/12)

(84) Designated Contracting States:
AT BE CH CY DE DK ES FI FR GB GR IE IT LI LU
MC NL PT SE TR
Designated Extension States:
AL LT LV MK RO SI

(30) Priority: 18.09.2000 JP 2000281207
08.03.2001 JP 2001065801
28.06.2001 JP 2001195916

(71) Applicant: I-Card Corporation
Tokyo 108-0071 (JP)

(72) Inventors:
• SUZUKI, Hideyuki
  Minato-ku, Tokyo 108-0071 (JP)
• NAKAMURA, Yusuke
  Yokohama-shi, Kanagawa 225-0011 (JP)

(74) Representative: Poulin, Gérard
Société BREVATOME
3, rue du Docteur Lancereaux
75008 Paris (FR)

(54) **MICRO WELL ARRAY AND METHOD OF SEALING LIQUID USING THE MICRO WELL ARRAY**

(57)     The invention offers a microwell array and sealing method thereof, wherein liquid is spotted in wells in an amount exceeding the volumes of the wells after welding, and the liquid is sealed by pushing the excess liquid from the wells so that almost no air remains inside the wells, so as to enable a reaction to be performed between minute quantities of a sample and reagent in a minuscule space, allowing for efficient extraction of fluorescent light which functions as a signal. The microwell array comprises a container having an array of a plurality of isolated wells, and a cover capable of covering the container, wherein a raised portion which is higher than the surrounding portions is formed in the peripheral portions of each well.

Fig. 1

## Description

TECHNICAL FIELD

The present invention relates to a microwell array used for sealing extremely minute quantities of a solution, a liquid sealing method using such a microwell array, a method for distributively injecting liquid into the microwell array, a manufacturing method for the microwell array, a welding apparatus for the microwell array, and an analysis method using the microwell array.

BACKGROUND ART

As one method for selecting desired compounds from among a number of compounds, there is HTS (High-Throughput Screening). HTS technology is capable of handling a large amount of biological information at once, and has recently gathered much attention for its overwhelming advantages in terms of cost and time over techniques in which chemical reactions and fluorescence detection are performed one test tube at a time.

For example, whereas DNA microarrays which are currently on the verge of coming into widespread use are used for the purpose of gene expression analysis, this is also a technology which arose from HTS technology, being an effective analysis means enabling the quantity of expressions to be compared and processed in parallel for each gene, by spotting probe cDNA (genes) among thousands to tens of thousands on the surface of a single glass slide, and performing hybridization with target cDNA obtained by reverse transcription of mRNA taken from a specimen.

In these DNA microarrays, the cDNA which is the target and the cDNA which is the probe are hybridized in a buffer solution, after which the slide is cleansed and dried, and the fluorescent light emitted from each spot measured by an optical scanner, but depending on the assay, there are often cases where the reaction and detection of signals must be performed in the state of a solution. For example, in the TaqMan PCR method, Invader method and RCA method used for SNP typing ("Strategies for SNP Genetic Polymorphism", edited by Yusuke Nakamura, pp. 93-149, Nakayama Shoten, June 2000), the reaction between the DNA and enzymes, as well as fluorescence detection after the reaction must be performed in the state of a solution.

While the Invader method is explained in V. Lyamichev et al., "Polymorphism identification and quantitative detection of genomic DNA by invasive cleavage of oligonucleotide probes", Nature Biotechnology 17 (1999), pp. 292-296, this method can be used to selectively detect polymorphism in genomic DNA. That is, as an experimental method, about 200 ng of genomic DNA are divided out, and this is mixed with 20 μL of a reagent (a mixed solution of fluorescent marker reagent + enzyme + Invader probe). Then, by measuring the intensity of the fluorescent light emitted from the solution, it is possible to determine whether or not the DNA sequence (so-called polymorphism) which is to be detected is present in the genomic DNA (called "typing").

Currently, when performing large numbers of solution reactions, it is normal to divide the reagent into a 96-well microtiter plate or a 384-well microtiter plate wherein each well has a volume of tens to hundreds of μL, and to perform a heat treatment in a thermal eyelet. During heating, it is necessary to seal the well holding the sample so that the liquid will not evaporate and be released from the well, and the well is usually covered by a flexible sheet or film coated with adhesive. In particular, a temperature of 95 °C close to the boiling point of water must be achieved for denaturation of DNA, a large pressure is applied on the sheet (or film) from inside the well. The well is completely sealed in order to keep solution which has evaporated from escaping from the well.

In order to perform such assays which include heat treatments efficiently, the material, shape, and physicochemical properties of 96-well microtiter plates and 384-well microtiter plates have been improved a number of times in accordance with the intended use. For example, PCT Application, Japanese First Publication No. H11-507508 describes an invention characterized by pressing flexible pads having resiliently contracting ridges formed on the surfaces of the pads against the openings of the wells when sealing the wells in a microarray, thereby holding the inside of the well in a liquid-proof state and enabling the pads to be readily peeled form the wells after the heating and stirring steps. US Patent No. 6,106,783 discloses a structure having the purpose of reducing cross-contamination when sealing the wells.

On the other hand, Japanese Patent Application, First Publication No. H10-221243 discloses a microplate wherein the side walls of the wells are formed of a non-transparent material in order to reduce the optical cross-talk between adjacent wells, and the bottom portions are formed of a material with high transparency in order to enable light emissions to be measured from above or below the wells. US Patent No. 5,487,872 discloses a microtiter plate having the bottom of the wells formed of a UV-transmitting material in order to enable light emissions to be measured easily from above or below the wells.

DISCLOSURE OF THE INVENTION

Since the enzymes (structurally proteins) used in the reactions and the fluorescent pigments used for detection of reaction products are extremely expensive, and the amount of genomic DNA capable of being extracted from a single sample is limited (100-200 μg of DNA can be collected from 20 cc of blood), thus making it difficult to withdraw a lot of information relating to DNA sequences, diseases and genes from small amount of samples. For this reason, assays wherein the amount of reagent and specimen in each well are made as small as possible by sealing minute amounts of solution in a tiny space and detecting light signals emitted from the solution at high sensitivities have been desired.

However, even if the well volumes of microtiter plates which have been conventionally used are simply made smaller, the solution in the wells cannot be incubated (thermal treatment) for long periods of time an isothermic bath unless the well sealing method itself is fundamentally changed. This is also clear from the fact that when water is heated to 95 °C and turned to steam, it becomes approximately 1600 times its volume at room temperature (25 °C), which means that nearly 1600 times the pressure is applied from inside the sealed well, the seal on the well cannot be maintained with a conventional sealing method.

The reduction of solution during a heat treatment in a thermal cycler can be suppressed to a minimum by the former invention for forming a liquid-proof seal of the solution. Additionally, it is possible to raise the quantity of fluorescence detected by means of the latter invention which has transparency in the bottom surface portion of the well. However, when performing a solution reaction such as a TaqMan method or an Invader method, tens to hundreds of μL of solution and hundreds of ng of genomic DNA must still be apportioned to each well even when using these inventions. Additionally, since the volume of the well in a microtiter plate is large, it is difficult to excite all of the fluorescent reagent in a well by means of light. Additionally, since the fluorescent light emitted from a well can be scattered by the side walls of wells with large volumes or be transmitted fro the bottom surface portion and dissipate, the fluorescent light cannot be detected at a high yield even if a plate reader is used. As a result, as long as a microplate is used, it is difficult to largely reduce the amount of fluorescent reagent, enzyme and sample, or to acquire large amounts of data in parallel.

Additionally, in recent years, technologies for isothermal amplification of DNA such as ICAN methods and Lamp methods have been developed, but as long as the well sealing methods of the conventional inventions are used, it is difficult to keep the wells liquid-proof for long periods of time in an isothermic bath.

The present invention has been conceived with the above considerations in mind, and has as its object to offer a microwell array which is capable of sealing reaction systems, which have conventionally been conceived as requiring at least 5.0 μL of reagent when using a microtiter plate for reaction or detection, in microwells in minute amounts of a few μL or less, and in some cases of 0.5 μL or less of solution, with almost no air being caught inside, these wells being arranged at a high density so as to be able to support reaction and detection of minute amounts of reagent, while treating them in parallel to extract large amounts of information. Additionally, it is possible to achieve microwell arrays of low cost by using those wherein the container and cover are composed of a plastic material.

According to an embodiment of the present invention, a microwell array comprises a container with a plurality of isolated wells positioned in an array, and a cover capable of covering the container; wherein raised portions which are higher than the surrounding portions are provided at the peripheral portions of each well. With this structure, it is possible to readily ensure a tight seal between the container and cover, making it suitable for sealing minute amounts of solution. Additionally, when the container and cover are to be welded by means of ultrasonic vibrations, the ultrasonic vibrations will be focused on the raised portions, thereby allowing for the weld to be readily accomplished. This is particularly suitable for the case in which DNA or proteins are contained in the fluid accommodated in the wells, because the weld can be performed without incurring any damage thereto. Furthermore, even if the fluid spills from the microwell when attaching the cover to the container and sealing, the raised portions prevent the spilled fluid from running into adjacent microwells and thereby causing cross-contamination.

According to another embodiment of the present invention, a microwell array comprises a container with a plurality of isolated wells positioned in an array, and a cover capable of covering the container; wherein raised portions for welding which are higher than the surrounding portions are provided at the peripheral portions of each well. With this structure, when the container and cover are to be welded by means of ultrasonic vibrations, the ultrasonic vibrations will be focused on the raised portions, thereby allowing for the weld to be readily accomplished.

According to another embodiment of the present invention, a microwell array comprises a container with a plurality of isolated wells positioned in an array, and a cover capable of covering the container; wherein raised portions which are higher than the surrounding portions are provided on the cover at positions corresponding to the peripheral portions of each well when the container is covered by the cover. In the case of this structure, as with the above-described structure, it is possible to readily ensure a tight seal between the container and cover, making it suitable for sealing minute amounts of solution. Additionally, when the container and cover are to be welded by means of ultrasonic vibrations, the ultrasonic vibrations will be focused on the raised portions, thereby allowing for the weld to be readily accomplished. This is particularly suitable for the case in which DNA or proteins are contained in the fluid accommodated in the wells, because the weld can be performed without incurring any damage thereto. Furthermore, even if the fluid spills from the microwell when attaching the cover to the container and sealing, the raised portions prevent the spilled fluid from running into adjacent microwells and thereby causing cross-contamination.

According to another embodiment of the present invention, a microwell array comprises a container with a plurality of isolated wells positioned in an array, and a cover capable of covering the container; wherein raised portions for welding which are higher than the surrounding portions are provided on the cover at positions corresponding to the peripheral portions of each well when the container is covered by the cover. In the case of this structure, as with the above-described structure, the presence of the raised portions provided on the cover enables the ultrasonic energy to be focused at these portions when welding the container and cover by means of ultrasonic vibrations, and is therefore favorable.

According to another preferable embodiment of the present invention, the raised portions are annular in the form of a circle, square or the like and thus have a shape which surrounds the wells, and their vertex portions are convex, not flat. This structure has the effect of reducing the size of the portion of contact between the container and cover where the vibrational energy is focused when bringing the container and cover into tight contact and welding them by ultrasonic vibrations, and is therefore favorable for enabling the welding to be more readily accomplished.

According to an aspect of the present invention, a microwell array comprises a container with a plurality of isolated wells positioned in an array, and a cover capable of covering the container; wherein channels for catching liquid overflowing from the wells when the wells are covered by the cover are formed in at least one of an area surrounding each well or the positions on the cover corresponding to said area surrounding each well. In order to prevent air from being trapped when injecting a test sample into a microwell and sealing, an extra portion of the test sample may spill from the microwells, but with the above structure, the fluid spilled from the wells can be received in the channels provided around the wells of the containers, thus preventing intermixture with fluid in adjacent wells which can result in cross-contamination. This structure is particularly effective when the container and cover are especially thin, and the thickness is still small even when they are combined.

According to another preferable embodiment of the present invention, the microwell array comprises a container with a plurality of isolated wells positioned in an array, and a cover covering the container; wherein said cover and said wells containing liquid are welded together at each well. By welding together the container and cover, the use of adhesives becomes unnecessary, so that the problem of elution of solvents does not occur, and the same level of strength as in the case where the container and cover have a unitary structure can be obtained at the bonded portions, as well as the container and cover being easier to handle prior to attachment.

According to another preferable embodiment of the present invention, the microwell array comprises a container with a plurality of isolated wells positioned in an array, and a. cover covering the container; wherein said cover and said wells containing liquid are ultrasonically welded together at each well. By welding together the container and cover by means of ultrasonic waves, the temperature is raised locally at only the welding portions without raising the temperature of the microwell array overall, and a liquid-tight seal can be achieved within a few seconds, which allows for a low cost and considerable increases in operability and productivity.

According to another preferable embodiment of the present invention, a microwell array comprises a container with a plurality of isolated wells positioned in an array, and a cover covering the container and sealing each well in a liquid-tight manner, wherein the substantial thicknesses of the container and cover are both of a thickness such as to be able to transmit the heat of liquid contacting said container and said cover to the insides of the wells. Here, "substantial thickness" refers to the thickness of the container or thickness of the cover at the portions constituting the wall surfaces defining the wells and not including the skirt portion or the like. With this structure, when the microwell array is immersed in an isothermic bath, the thickness is such that thermal energy is efficiently transmitted from the surrounding liquid to the insides of the wells, thus enabling incubation to be performed effectively.

According to another preferable embodiment of the present invention, a microwell array comprises a container with a plurality of isolated wells positioned in an array, and a cover covering the container and sealing each well in a liquid-tight manner, wherein the substantial thicknesses of the container and cover are both within the range of 0.15-3.0 mm. By making this thickness 0.15-3.0 mm, the heat of a water bath or thermal cycler can be effectively conducted into the wells, while providing enough strength so as not to be damaged when handling.

According to another preferable embodiment of the present invention, the microwell array comprises a container with a plurality of isolated wells positioned in an array, and a cover covering the container; wherein the substantial thickness of the microwell array when said well and said cover are welded in a liquid-tight manner at each well is within the range of 0.3-4.0 mm. Here, "substantial thickness" refers to the thickness from the top surface of the cover to the bottom surface of the container in the vicinity of the wells and not including the skirt portions. By setting this thickness to 0.3-4.0 mm, the heat can be effectively transmitted inside the wells after welding the cover and container, while providing enough strength so as not to be damaged when handling.

According to another preferable embodiment of the present invention, a microwell array comprises a container

with a plurality of isolated wells positioned in an array, and a cover covering the container; wherein the thickness of the portions directly defining the wells when said well and said cover are welded in a liquid-tight manner at each well is within the range of 0.05-0.4 mm. Due to this structure, even if the thickness at other portions is large, heat can be effectively transmitted inside the wells from the portions having a thickness of 0.

According to another preferable embodiment of the present invention, a microwell array comprising a container with a plurality of isolated wells positioned in an array, and a cover covering the container and sealing each well in a liquid-tight manner, wherein welding ribs having sufficient volume for welding said well s and said cover are provided in the vicinity of said wells. By setting the cross section of these ribs to a sufficiently large area in order to ensure strength after welding, it is possible to maintain the seal for each well.

According to another preferable embodiment of the present invention, a microwell array comprises a container with a plurality of isolated wells positioned in an array, and a cover covering the container and sealing each well in a liquid-tight manner, wherein welding ribs having a triangular cross-section are provided in the vicinity of said wells. By setting the bases of the ribs, that is, the width of the ribs prior to welding to the above range, the load on the output of the ultrasonic welding device can be reduced, thus enabling welding under stable conditions without overload.

According to another preferable embodiment of the present invention, a microwell array comprises a container with a plurality of isolated wells positioned in an array, and a cover covering the container and sealing each well in a liquid-tight manner, wherein the thicknesses of the bottoms of welding ribs in the vicinity of said wells are within the range of 0.2-1.0 mm. By setting the bases of the ribs, that is, the width of the ribs prior to welding to the above range, the load on the output of the ultrasonic welding device can be reduced, thus enabling welding under stable conditions without overload.

According to another preferable embodiment of the present invention, a microwell array comprises a container with a plurality of isolated wells positioned in an array, and a cover covering the container and sealing each well in a liquid-tight manner, wherein the thicknesses of the bottoms of welding ribs in the vicinity of said wells are within the range of 0.2-1.0 mm, their heights are within the range of 0.2-0.8 mm, and the diameters of ribs surrounding said wells are within the range of 0.5-4.0 mm. Due to this structure, the load on the ultrasonic welding device can be reduced, and by setting the height and diameter of the ribs to the above range, variance in the height of the ribs can be absorbed, moreover without adding any load to the welding process.

According to another preferable embodiment of the present invention, a microwell array comprising a container with a plurality of isolated wells positioned in an array, and a cover covering the container and sealing each well in a liquid-tight manner, wherein the widths of welding portions surrounding the wells for joining said wells and said cover at each well in a liquid-tight manner are within the range of 0.3-2.5 mm. According to this structure, even if the liquid inside the wells boils after welding, it is possible to sufficiently maintain the tightness of the seal between the cover and the container.

According to another embodiment of the present invention, a microwell array comprises a container with a plurality of isolated wells positioned in an array, and a cover capable of covering the container; wherein said wells have a capacity such that the liquid temperature inside said wells becomes uniform within a few minutes upon immersion of said microwell array in an isothermic bath. By making the capacity such that the heat is effectively and uniformly transmitted, it is possible to efficiently induce a chemical reaction inside the well.

According to another preferable embodiment of the present invention, a microwell array comprising a container with a plurality of isolated wells positioned in an array, and a cover capable of covering the container; wherein the capacity of said wells is within the range of 0.1-1.4 μl. According to another preferable embodiment of the present invention, a microwell array comprises a container with a plurality of isolated wells positioned in an array, and a cover capable of covering the container; wherein the capacity of said wells when said wells and said cover are joined in a liquid-tight manner at each well is within the range of 0.1-1.4 μl. By making the well capacity such as to be within this range, the capacity of the wells can be reduced to the measurable limit, thus enabling a large number of samples to be handled.

According to a preferable embodiment of the present invention, a microwell array comprises a container with a plurality of isolated wells positioned in an array, and a cover covering the container; wherein said well and said cover seal the wells in a liquid-tight manner, and the seal of the wells is maintained even if the liquid boils inside the wells. The tight seal achieved by the present invention has a strength which was not achievable by conventional sealing methods using adhesives. For this reason, the microwell array can be directly processed at high temperatures without any mechanical seal aiding means.

According to another preferable embodiment of the present invention, a microwell array comprises a container with a plurality of isolated wells positioned in an array, and a cover covering the container; wherein after the wells and the cover are welded in a liquid-tight manner by irradiation with ultrasonic waves, the seal on the wells is maintained even if liquid boils inside the wells. That is, in the present embodiment, the above-described seal is achieved by welding together the container and cover by ultrasonic waves.

According to another preferable embodiment of the present invention, a microwell array comprises a container with a plurality of isolated wells positioned in an array, and a cover covering the container; wherein said well and said cover seal the wells in a liquid-tight manner, and the seal of the wells is maintained without applying any external mechanical forces even if the liquid boils inside the wells. That is, while conventional microwell arrays are capable of holding a certain degree of tightness of the seal under atmospheric pressure, the seal would break under stringent conditions such as when the liquid inside the well portions boiled, while the microwell array of the present invention has a structure wherein the wells themselves can withstand the internal pressure due to improvements in the sealing structure.

According to another embodiment of the present invention, a microwell array comprises a container with a plurality of isolated wells positioned in an array, and a cover covering the container; wherein said well and said cover seal the wells in a liquid-tight manner, and the seal of the wells is maintained even if a liquid heated to 90-100 °C boils inside the wells. That is, the temperature at which the liquid inside the wells as described above, is specifically 90-100 °C, and the microwell array of the present invention is capable of holding the boiling pressure of the liquid inside the wells at these temperatures.

According to another preferable embodiment of the present invention, microwell array comprising a container with a plurality of isolated wells positioned in an array, and a cover covering the container; wherein said well and said cover seal the wells in a liquid-tight manner, and the seal of the wells is maintained even if said microwell array is immersed in a boiling liquid

According to yet another embodiment of the present invention, the microwell array comprises an intermediary body which is roughly planar and composed of a material having flexibility is placed between the container and the cover. In the case of the microwell array of this structure, the intermediary body is flexible, so that when the cover is pressed against the container for the seal, the intermediary body can be easily brought into tight contact with the top portion of the microwells. Additionally, due to the intermediary body contacting the top edge portion of the microwells and deforming, the air inside the microwells can be expelled, which is favorable.

According to yet another embodiment of the present invention, a microwell array comprises a container with a plurality of isolated wells positioned in an array, and a cover capable of covering the container; wherein a convex portion which is pushed into each well when sealing the well by means of the cover is formed at a position of the cover corresponding to each well when the container is covered by the cover. In the case of the microwells having this structure, when the cover is pressed against the container, the convex portions are pressed into the wells, as a result of which air bubbles which may be present inside the wells are expelled from the wells along with some liquid depending on the case, which is favorable.

According to another embodiment of the present invention, a liquid sealing method comprises steps of using a container having a plurality of isolated wells positioned in an array and a cover capable of covering the container, injecting liquid into the wells, pressing the cover onto the container, then welding together said cover and said container to seal said liquid into each well. According to this method, the work efficiency is improved because the tightening operation and the sealing operation due to welding can be performed simultaneously, and further, contamination of the liquid inside the wells can be prevented because no adhesive is used.

According to another embodiment of the present invention, the liquid sealed into the wells as described above is a liquid containing DNA or proteins. In this case, the container and cover are welded together, so that the liquid can be sealed while reliably prevented from contacting the outside air, and the result is convenient to handle after sealing.

According to yet another embodiment of the present invention, the microwell array with liquid containing DNA or proteins sealed into the wells is such that the container and cover are welded by means of ultrasonic waves. By focusing the vibrational energy due to the ultrasonic vibrations at the welding portion, the seal can be accomplished without damaging the DNA or proteins, so as to obtain a seal without sacrificing the effectiveness of the DNA or proteins.

According to yet another preferable embodiment of the present invention, the ultrasonic wave radiation for welding the container and cover is selected from among those which are sufficient for welding while simultaneously substantially not damaging the DNA or proteins. In the present specification, substantially not damaging DNA or proteins refers to the case where enough DNA or proteins remain in the wells to enable subsequent analysis. By appropriately selecting the radiation energy of the ultrasonic waves from the above-given range, it is possible to obtain a sealing method which enables a seal which is most suitable for analysis.

According to yet another embodiment of the present invention, the wells of said container and said cover are welded by ultrasonic vibrations lasting 0.05 to 0.8 seconds for a liquid-tight seal. By performing ultrasonic welding under these conditions, it is possible to ensure a reliable seal, while also reducing the ultrasonic energy.

According to yet another embodiment of the present invention, the vibration of the ultrasonic horn is started while applying a force of 0.3 to 100 N per 1 cm of length of a raised portion to be welded. By performing ultrasonic welding under these conditions, it is possible to ensure a reliable seal, while also reducing the ultrasonic energy.

According to yet another embodiment of the present invention, the container and said cover are composed of materials capable of sealing off each well by means of ultrasonic welding. According to this structure, the container and cover can readily be ultrasonically welded without affecting the test sample accommodated inside the wells.

According to yet another embodiment of the present invention, the microwell has the further characteristic that the rear surface of each well is planar. With this structure, the microwell array can be placed on a flat heat block for heating. Additionally, a plurality of microwell arrays can be stacked, and they also become easier to mold.

According to yet another embodiment of the present invention, a reflective surface for reflecting light is provided on the inner wall surfaces of the wells or on the bottom surface of the cover of the microwell array. Due to this structure, by measuring the fluorescent light with the reflecting surface as the backdrop, the amount which is measured increases, thus substantially improving the measuring sensitivity. Furthermore, when the inside wall surfaces of the wells are given a reflective surface, the leakage of fluorescent light to adjacent wells is also prevented, thereby reducing cross-talk.

According to yet another aspect of the present invention, a liquid injecting method using a microwell array as described above, comprising steps of first distributively injecting a liquid by means of a contact-type distributive injector, then distributively injecting a liquid by means of a non-contact-type distributive injector is offered. With this method, it is possible to make use of the advantages of both high-speed distributive injection by a contact type distributive injector and distributive injection without cross-contamination due to a non-contact type distributive injector.

According to another embodiment of the present invention, the method comprises steps of first distributively injecting a different liquid in each well by means of a contact-type distributive injector, then distributively injecting a liquid by means of a non-contact-type distributive injector. Due to this method, the distributively injected liquid is injected by means of non-contact type distributive injection, as a result of which cross-contamination will not occur.

According to yet another embodiment of the present invention, the method comprises steps of first distributively injecting liquid by means of a contact-type distributive injector, drying said liquid, then distributively injecting a liquid by means of a non-contact-type distributive injector. Due to this method, contamination will not occur even if different liquids are distributively injected multiple times. Furthermore, the liquid is distributively injected by non-contact type distributive injection, as a result of which cross-contamination will not occur.

According to yet another embodiment of the present invention, the wells of the microwell array have a circular horizontal cross section. In the case of this structure, the possibility of air bubbles adhering to the inner wall surface of the wells is small, and the vibrational energy to be irradiated to obtain a uniform distribution of the vibrational energy when sealing the upper surface by ultrasonic welding is reduced.

According to yet another embodiment of the present invention, a skirt portion is formed along the outer peripheral portion of said microwell array. Due to this structure, the outward shape of the microwell array can be made roughly box-shaped or planar, thus making it easier to handle in case of stacking or the like.

According to yet another embodiment of the present invention, through holes are formed in the corners of the skirt portion formed on said microwell array. Due to this structure, positioning is made easier because the through holes formed in the skirt portion can be used as standard positions for alignment when injecting liquid or measuring the fluorescent light.

According to yet another embodiment of the present invention, an insertion portion and receiving portion are formed on said container and said cover, and said container and said cover can be engaged by fitting the insertion portion into the receiving portion. Due to this structure, the cover and container can be readily attached, and their positional alignment is also made easier.

According to yet another embodiment of the present invention, said container and said cover of the microwell array are formed of a plastic material. Due to this structure, the production cost for the microwell array can be reduced due to unitary molding, and it is compatible with welding due to ultrasonic waves.

According to yet another embodiment of the present invention, said container and said cover of the microwell array are composed of a methyl pentene copolymer or polycarbonate, whereby unitary molding and welding by ultrasonic waves is made possible, and the fluorescent light measurements can be performed efficiently due to the high transparency.

According to yet another embodiment of the present invention, at least one of said container and said cover of the microwell array is formed of an optically transparent material. Due to this structure, the fluorescent light can be efficiently measured.

According to another embodiment of the present invention, a liquid reagent or sample is distributively injected and held in the wells of said container or on the surface on the well side of said cover forming the microwell array. Due to this structure, the reagent or sample, or their combination accommodated in each well can be independently controlled.

According to another embodiment of the present invention, a liquid reagent or sample is distributively injected by a non-contact-type distributive injector. Due to thereto, the possibility of cross-contamination of reagent and samples between wells can be largely reduced.

According to another embodiment of the present invention, at least one of a reagent or sample is held in the wells of said container, the other is held on the surface of the cover, and said wells and said cover are joined by ultrasonic welding to induce a reaction between the reagent and sample in each well. According to this method, the reagent and sample first contact each other during ultrasonic welding, which is extremely favorable for the case where a reagent and sample which are preferably held separate prior to mixing are to be combined. Additionally, according to this method, distributive injection can be performed by only a contact type distributive injector, thus reducing the possibility of cross-contamination.

Additionally, according to another aspect of the present invention, a microwell array is produced by pouring resin in from a side gate for injection molding of said container and said cover. This production method is a suitable method for achieving the production precision and flatness which are needed in the microwell array according to the present invention.

According to another aspect of the present invention, an ultrasonic welding apparatus for performing the ultrasonic welding of said container and cover is capable of applying a force of 7000-23000 N during oscillation, and has a maximum oscillation output of 4.1-5.0 kW. In order to melt and weld together the raised portions around the wells where the container and cover achieve contact by means of vibrations, the performance of the ultrasonic welding apparatus must exceed that of conventional equipment.

According to another embodiment of the ultrasonic welding apparatus of the present invention, the horn amplitude is 30-40 microns, and it is capable of applying a force of 7000-23000 N during oscillation, having a maximum oscillation output of 4.1-5.0 kW. By using a horn having these properties, it is possible to achieve a strong seal between the container and cover which is the object of the present invention with respect to the microwell array.

According to another embodiment of the ultrasonic welding apparatus of the present invention, the horn amplitude is 30-40 microns, and it is capable of applying a force of 7000-23000 N during oscillation, having a maximum oscillation output of 4.1-5.0 kW, and being capable of emitting ultrasonic waves within a welding time of 0.05-0.8 seconds. While the hom must be capable of radiating the ultrasonic vibrational energy needed for welding the container and cover without at the same time destroying the molecules of the object of measurement, these requirements are satisfied by a hom having the above-described conditions.

According to another embodiment of the ultrasonic welding apparatus of the present invention the horn amplitude is 30-40 microns, and is capable of applying a force of 7000-23000 N during oscillation, having a maximum oscillation output of 4.1-5.0 kW, and is capable of welding each well within a time of 0.05-0.8 seconds. According to this ultrasonic welding apparatus, the ultrasonic vibrational energy is spread over the wells in order to weld the wells.

According to another aspect of the present invention, a sealing and distributive injecting method, comprising steps of distributively injecting a reagent or sample into well portions or cover surfaces of a microwell array comprising a container having a plurality of isolated wells arranged in an array, and a cover capable of covering the container, then welding together said cover and said wells so that each well is liquid-tight is proposed. By performing this sealing and welding method, it is possible to firmly seal the reagent or sample accommodated in each well while holding the possibility of cross-contamination to a minimum.

According to another aspect of the present invention, an analysis method comprising steps of distributively injecting a reagent or sample into well portions or cover surfaces of a microwell array comprising a container having a plurality of isolated wells arranged in an array, and a cover capable of covering the container, then welding together said cover and said wells so that each well is liquid-tight, and performing fluorescent light intensity analysis for each well after enabling the reagent and sample to react, or while enabling the reagent and sample to react is proposed. By means of the this method, the level of progress of a reaction can be analyzed with high precision using extremely small amounts of reagent or sample.

According to another aspect of the present invention, a genetic analysis method comprising steps of distributively injecting a reagent or sample into well portions or cover surfaces of a microwell array comprising a container having a plurality of isolated wells arranged in an array, and a cover capable of covering the container, then welding together said cover and said wells so that each well is liquid-tight, and performing fluorescent light intensity analysis for each well after enabling the reagent and sample to react, or while enabling the reagent and sample to react, thereby to analyze the genes of each well is offered. Due to this method, it is possible to perform genetic analysis with high precision using very small amounts of reagent or sample.

According to another aspect of the present invention, a genetic polymorphism analysis method comprising steps of distributively injecting a reagent or sample into well portions or cover surfaces of a microwell array comprising a container having a plurality of isolated wells arranged in an array, and a cover capable of covering the container, then welding together said cover and said wells so that each well is liquid-tight, and performing fluorescent light intensity analysis for each well after enabling the reagent and sample to react, or while enabling the reagent and sample to react, thereby to analyze the genetic polymorphism of each well is offered. Due to this method, it is possible to perform genetic polymorphic analysis with high precision using very small amounts of reagent or sample.

According to another preferable embodiment of the present invention, a genetic polymorphism analysis method comprising steps of distributively injecting different DNA into each well of a microwell array comprising a container having a plurality of isolated wells arranged in an array, and a cover capable of covering the container, next distributively injecting reagent into the plurality of said wells, then welding together said wells and said cover, enabling the reagent to react with the DNA, and analyzing the fluorescent light intensity of each well to perform polymorphic typing is offered. By using this analysis method, genetic polymorphism analysis can be readily performed through analysis of fluorescent light intensity with only a small amount of solution.

According to another preferable embodiment of the present invention, a genetic polymorphism analysis method comprising steps of distributively injecting a reagent into each well of a microwell array comprising a container having a plurality of isolated wells arranged in an array, and a cover capable of covering the container, next distributively injecting different DNA into the plurality of said wells, then welding together said wells and said cover, enabling the reagent to react with the DNA, and analyzing the fluorescent light intensity of each well to perform polymorphic typing is offered. By using this analysis method, genetic polymorphism analysis can be readily performed through analysis of fluorescent light intensity with only a small amount of solution.

According to another preferable embodiment of the present invention, genetic polymorphism analysis method comprising steps of distributively injecting a reagent a cover surface of a microwell array comprising a container having a plurality of isolated wells arranged in an array, and a cover capable of covering the container, next distributively injecting different DNA into the plurality of said wells, then welding together said wells and said cover, enabling the reagent to react with the DNA, and analyzing the fluorescent light intensity of each well to perform polymorphic typing. By using this analysis method, genetic polymorphism analysis can be readily performed through analysis of fluorescent light intensity with only a small amount of solution.

According to another preferable embodiment of the present invention, a genetic diagnosis method comprising steps of distributively injecting different reagents onto a cover surface of a microwell array comprising a container having a plurality of isolated wells arranged in an array, and a cover capable of covering the container, next distributively injecting different DNA into the plurality of said wells, then welding together said wells and said cover, enabling the reagent to react with the DNA, and analyzing the fluorescent light intensity of each well to perform genetic polymorphism analysis is offered. By using this analysis method, genetic diagnosis can be readily performed through analysis of fluorescent light intensity with only a small amount of solution.

According to another preferable embodiment of the present invention, a genetic diagnosis method comprising steps of distributively injecting different reagents into the wells of a microwell array comprising a container having a plurality of isolated wells arranged in an array, and a cover capable of covering the container, next distributively injecting different DNA into the plurality of said wells, then welding together said wells and said cover, enabling the reagent to react with the DNA, and analyzing the fluorescent light intensity of each well to perform genetic polymorphism analysis is offered. By using this analysis method, genetic diagnosis can be readily performed through analysis of fluorescent light intensity with only a small amount of solution.

According to another aspect of the present invention, an analysis method comprising steps of appending a bar code corresponding to each reagent and sample distributively injected into a microwell array comprising a container having a plurality of isolated wells arranged in an array, and a cover capable of covering the container, enabling the progress to be managed by the bar code for each step or each microwell array, then welding together said cover and said wells so that each well is liquid-tight, and performing fluorescent light intensity analysis for each well after enabling the reagent and sample to react, or while enabling the reagent and sample to react, thereby to analyze at least one of the degree of the reaction, genes and genetic polymorphism for each well is offered. According to the present method, the data can be more conveniently handled when performing analysis using multiple or many types of reagents or the like, thus reducing the possibility of mistakes due to error.

According to yet another embodiment of the present invention, at least one of said container or said cover is produced by injection molding by pouring resin from a side gate. By employing this production method, it is possible to obtain a high degree of flatness without warpage even if the thickness of the container or cover is small.

According to another aspect of the present invention, a liquid sealing method using a microwell array such as described above, wherein liquid is distributively injected into the wells, and the cover or intermediary body is pressed against the container so as to push liquid out from the wells, thereby sealing liquid into the wells while preventing intermixture of air into the wells is offered. By sealing the liquid using this method, the intermixture of air into the wells can be avoided, and increases in internal pressure in the wells due to expansion of the air in subsequent high temperature processing can be prevented.

According to yet another embodiment of the present invention, the welding is performed by ultrasonic welding. Due to the ultrasonic welding, the wells can be sealed while minimizing the effect of contamination or the like on samples contained in the wells. In particular, it is possible to reduce the energy required for ultrasonic welding by appropriately selecting the shape of the portions of contact between the container and cover, that is, the peripheral portions or cover-contacting portions of the wells, thereby suppressing the influence on the liquid to such as degree as to be able to substantially ignorable.

The above gives examples of possible means offered by the present invention and their effects, and the effects of the above-described means aside from the above, and the effects obtained by means offered by the present invention other than those given above should be capable of being readily understood by those skilled in the art based on the description of the embodiments given below.

BRIEF DESCRIPTION OF THE DRAWINGS

Fig. 1 is a perspective view for explaining a microwell array of the present invention.
Fig. 2 is a section view for explaining a microwell array of the present invention.
Fig. 3 is a section view for explaining another microwell array of the present invention.
Fig. 4 is a section view for explaining a fitting portion of the present invention.
Fig. 5 is a section view for explaining a liquid runoff channel of the present invention.
Fig. 6 is a section view for explaining a liquid runoff channel of the present invention.
Fig. 7 is a diagram for explaining a method of positioning a reflective body in the present invention.
Fig. 8 is a diagram for explaining a method of positioning a skirt portion, a gate position and a through hole in the present invention.
Fig. 9 is a diagram for explaining a liquid sealing method of the present invention.
Fig. 10 is a diagram for explaining another liquid sealing method of the present invention.
Fig. 11 is a diagram for explaining a distributive injection method of the present invention.
Fig. 12 is a diagram for explaining the relationship between ultrasonic welding time and reactivity.
Fig. 13 is a diagram for explaining the relationship between the vibration force and remaining liquid amount.
Fig. 14 is a diagram for explaining a gene analysis method according to the present invention.
Fig. 15 is a perspective view for explaining another microwell array of the present invention.
Fig. 16 is a section view for explaining a microwell array of the present invention.
Fig. 17 is a diagram for explaining another liquid sealing method of the present invention.
Fig. 18 is a diagram for explaining a recessed portion for adhesive and a raised portion for ultrasonic welding of the present invention.
Fig. 19 is a diagram for explaining a recessed portion for adhesive or a raised portion for ultrasonic welding of the present invention.
Fig. 20 is a diagram for explaining a fitting portion and a raised portion for ultrasonic welding of the present invention.
Fig. 21 is a diagram for explaining another liquid sealing method of the present invention.
Fig. 22 is a diagram for explaining another liquid sealing method of the present invention.
Fig. 23 is a diagram for explaining another liquid sealing method of the present invention.
Fig. 24 is a diagram for explaining a typing method and a genetic diagnosis method according to the present invention.
Fig. 25 is a perspective view of an embodiment of a microwell array according to the present invention having 384 microwells and channels between the microwells.
Fig. 26 is a diagram showing the embodiment of the microwell array shown in Fig. 25 as seen from above.
Fig. 27 is a side view of the microwell array shown in Fig. 25.
Fig. 28 is another side view of the microwell array shown in Fig. 25.
Fig. 29 is a bottom view of the microwell array shown in Fig. 25.
Fig. 30 is a vertical section view of the microwell array along AA in Fig. 25.
Fig. 31 is a vertical section view of the microwell array along BB in Fig. 25.
Fig. 32 is an enlarged section view of the portion indicated by CC in Fig. 30.
Fig. 33 is a drawing showing the bottom surface of the lid of the microwell array shown in Fig. 25.

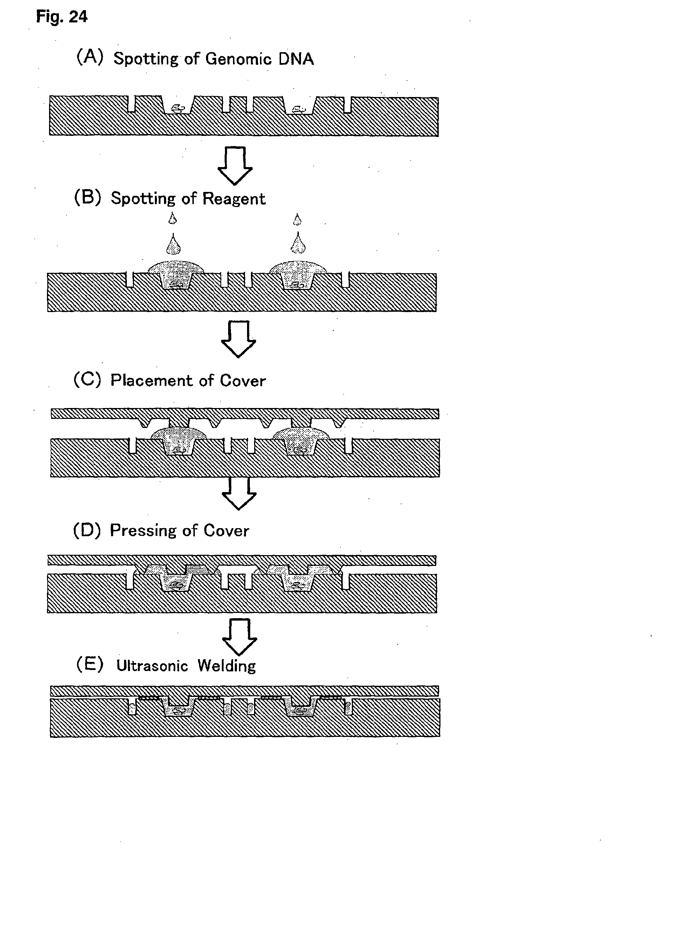

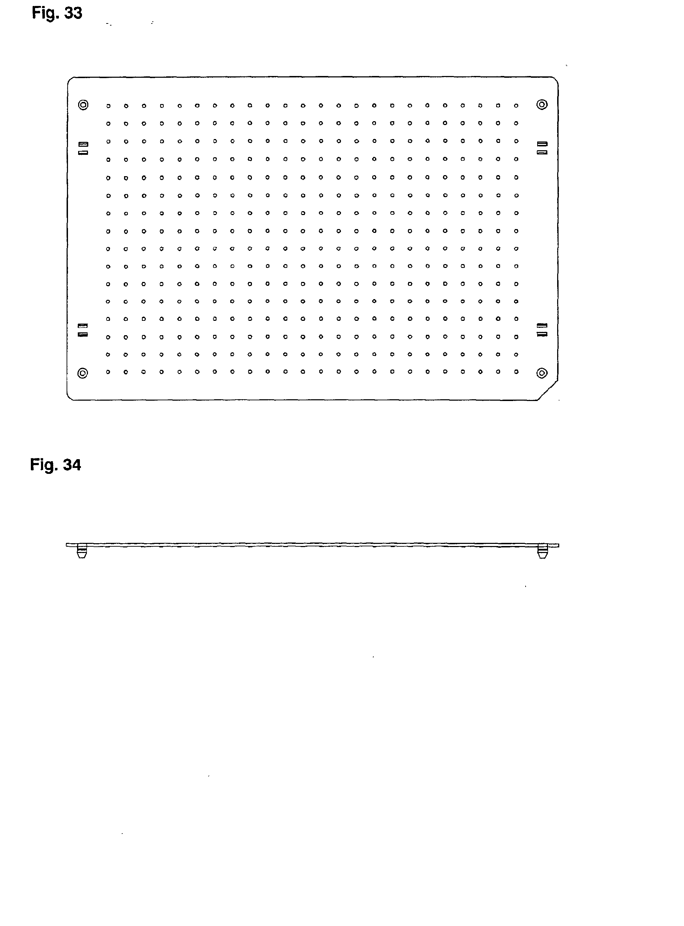

Fig. 24 is a side view showing the lid shown in Fig. 33 as seen from the side.

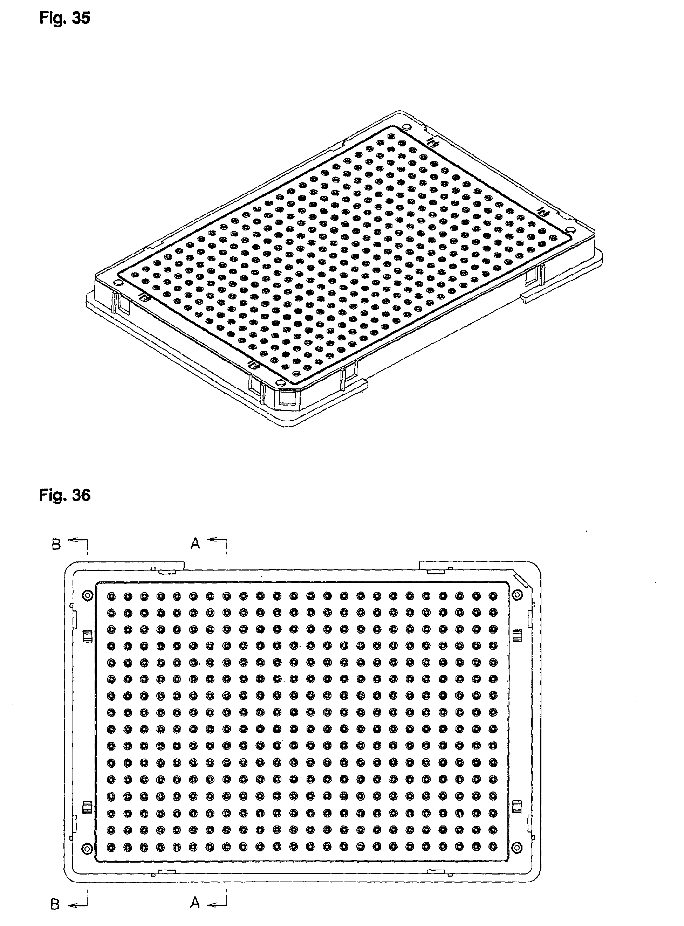

Fig. 35 is a perspective view showing an embodiment of the present invention having 384 microwells but without the channels between the microwells.
Fig. 36 is a drawing showing the embodiment of the microwell array shown in Fig. 35 as seen from above.

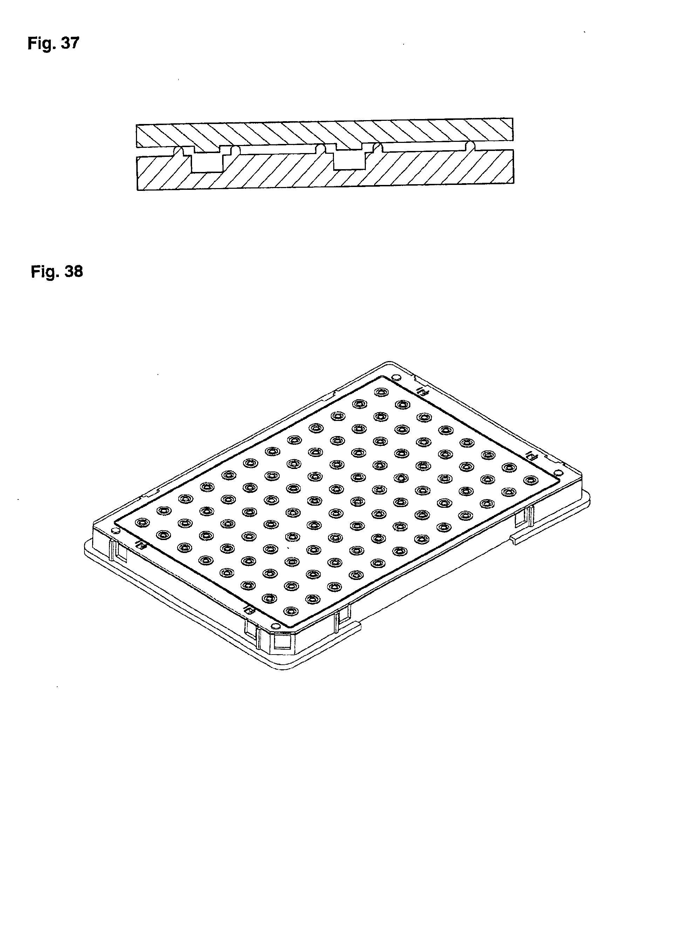

Fig. 37 is a partially enlarged section view of the microwell array shown in Fig. 36.
Fig. 38 is a perspective view of a microwell array according to an embodiment of the present invention having 96 microwells.

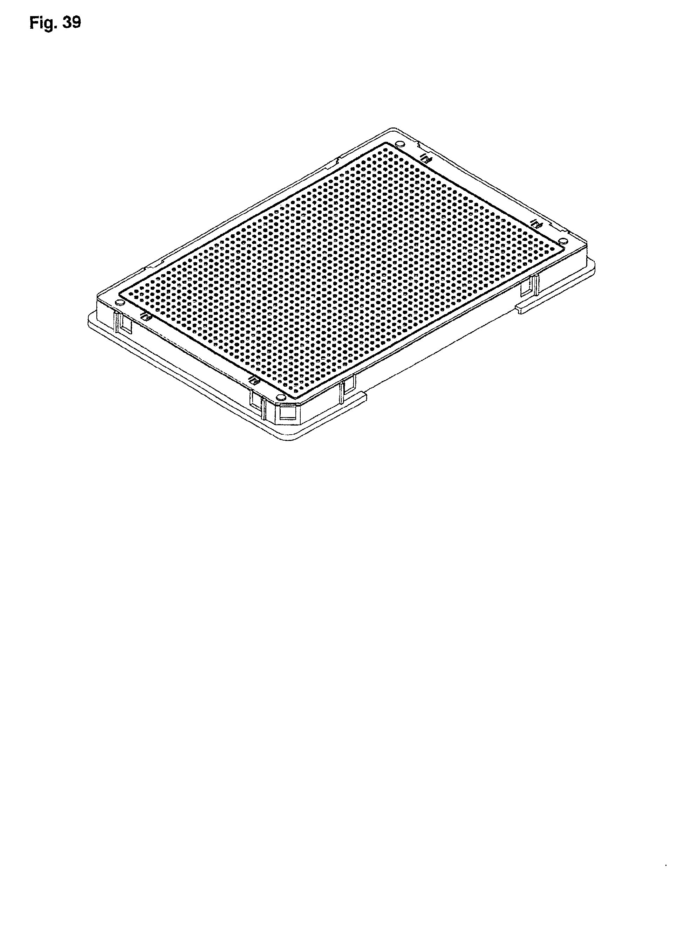

Fig. 39 is a perspective view of a microwell array according to an embodiment of the present invention having an extremely large number of microwells.

BEST MODES FOR CARRYING OUT THE INVENTION

Herebelow, preferred embodiments of the present invention shall be described with reference to the drawings.
Until now, ultrasonic waves have been used for the purpose of destroying DNA and cells. For example, the shotgun method wherein DNA are pulverized by means of ultrasonic waves to form short fragments, then the base sequence read by a sequencer is a good example. Additionally, the destruction of cell membranes by applying ultrasonic waves to cells has also been attempted. However, as has been proposed in the present invention, it has become clear that ultrasonic technologies which have been used for the purposes of destruction until now can be used to seal small amount of solution, DNA and proteins by modifying the structure of the containers or the welding conditions due to ultrasonic waves. Thus, the present invention is extremely significant for having put into practice an application which was heretofore unthinkable.

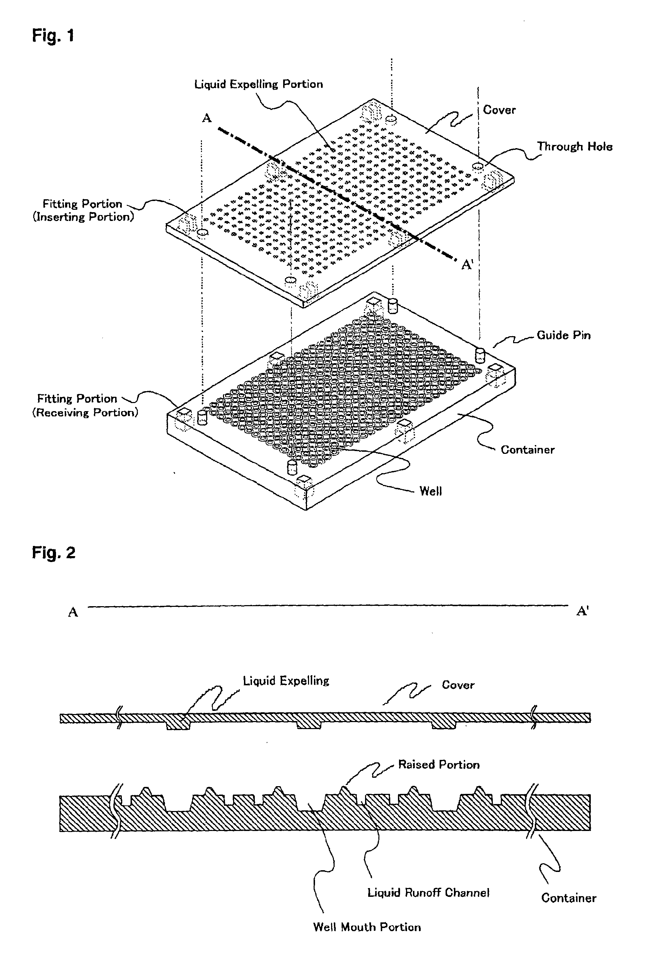

Fig. 1 is a perspective view of the microwell array of the present invention. In this example, 384 isolated.wells are formed on the surface of a plastic container at a lateral spacing of 4.5 mm. Here, "isolated" refers to a state of being completely separated in such a way that there will be no mixture of the liquid in different wells. By pressing the cover toward the well from above, it is possible to make a liquid-proof seal of a minute amount of liquid filling the well such that almost no air is mixed in. Additionally, through holes and guide pins are provided at the four corners in order to precisely align the positions of the cover and container, and a fitting portion is formed as shown in the drawing so that the cover and container are readily joined when pressed together. Fig. 2 is a section view along the line A-A' of Fig. 1, and as in the drawing, the well is formed from a mouth portion of the well, a raised portion and a liquid runoff channel. Here, the bump portion is a portion which is melted and adheres when welding the cover and the container.

In general, methods of adhering plastic materials together or to other materials include welding, solvent-based adhesion and adhesives. Welding is a method wherein plastics are made to adhere by thermal fusion, including the external heating type (gas pot jet, heat sealing, infrared, impulse sealing methods) and internal heating type (high-frequency welders, stitching, microwaves, ultrasonic sealing methods). Additionally, the vibration welding method of welding by means of vibrations is also included in welding. In a preferred embodiment of the present invention, the cover and container are welded by ultrasonic waves by means of an ultrasonic welder, but it is of course possible also to use vibration welding, solvents or adhesives.

The vertical cross sections of the wells are trapezoidal in order to make them easier to find for the needles of a spotting apparatus for injecting minute amounts of liquid. There will be cases in which there is not enough space to allow for a trapezoid if the wells are provided at a high density (e.g. 9600 wells), in which case the cross section may be rectangular. Accordingly, it is necessary to determine the optimum cross section, which may be polygonal such as triangular or quadrangular, or may be semicircular, according to the density of the wells and the size and shape of the needles.

While the liquid expelling portion (bump portion) is for pushing the liquid filling the wells out to the runoff channels so as not to leave any air in the wells, if the liquid which completely fills the wells sticks out from the mouth portions of the wells and bulges due to the surface tension, it is possible to seal the liquid so as not to leave air even if liquid expelling portions are not formed. Should a large amount of air remain in the well, and there be air bubbles in the well even after sealing, not only will the light be scattered, but the amount of the solution emitting light will be reduced, as a result of which the amount of fluorescent light emitted from the solution would decrease. Additionally, if the intensity of fluorescent light emitted from wells in which air bubbles are present and wells in which they are not present differs largely, there will be variance between signals of different wells, so that not only quantitative analysis, but even qualitative analysis will become difficult. Therefore, it is important to seal the solution in such a way that as few air bubbles are left in the wells as possible.

The liquid expelling portion also has in addition to the above function, the effect of keeping the raised portion fused at the time of welding from blocking the portion above the wells. That is, when the raised portion melts, the resin forming the raised portion will melt out and spread between the container and the cover, but the resin which has melted out in this way can be prevented from covering the tops of the wells by forming this liquid expelling portion, which is extremely effective for maintaining light transmissivity.

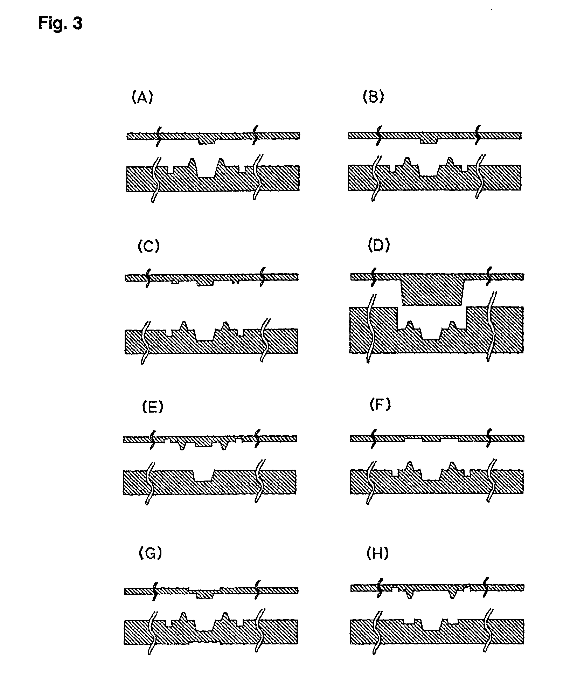

Additionally, in this example, raised portions for concentrating the energy of the ultrasonic waves are formed on the surface of the container, but similar effects can be expected if they are provided on the surface of the cover. Fig. 3 shows other examples of the vertical cross section around the wells. Fig. 3(A) shows the case where the shoulder portion of the raised portion formed around the well is connected with a portion of the well. In this state, not only will a portion of the raised portion which is melted at the time of welding melt out from the well and reduce the volume of the well, but there may be cases in which the liquid in the wells is heated due to melting out. Furthermore, there may be cases where the liquid in the wells absorbs a portion of the frictional heat which is supposed to be collected in the raised portion, thus making it difficult for the cover and well to be welded. Accordingly, it is more preferable for the shape of the vertex of the raised portion in the area around the well to have a convex shape as in Figs. 3(B)-(H).

Furthermore, the raised portion formed around the wells or on the cover should preferably have a circular or rectangular annular shape, that is, a shape which surrounds the well, the vertex portion being convex and not flat.

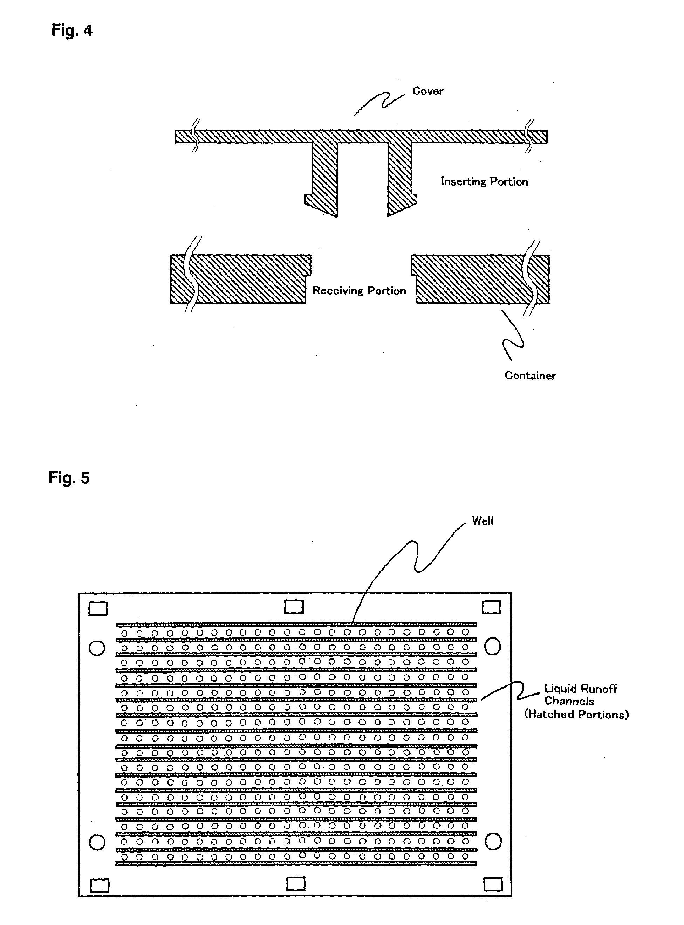

Additionally, Fig. 4 shows the cross section of a fitting portion. By working plastic to have a hook shape as in Fig. 4, it is possible to snap the cover into the container by bending the hook portion inward.

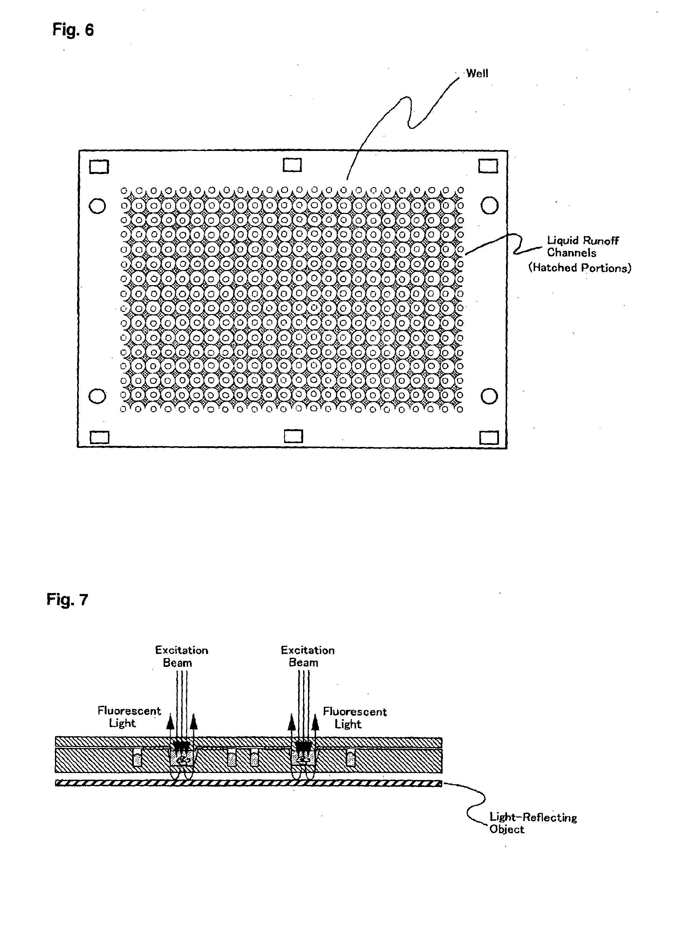

While the above-described liquid runoff channels are formed so as to surround the mouth portions of the wells, their shape and position need not be restricted as long as they are channels in which liquid expelled from the wells can collect. For example, it is possible to form runoff channels in straight lines between columns of wells as shown in the plan view of Fig. 5, or to form them in positions surrounded by four wells as shown in Fig. 6. Additionally, similar effects can be expected if they are provided on the surface of the cover.

In the above examples, the outer shape of the wells is designed to be circular so as to apply the energy of the ultrasonic waves in a uniform manner, but the outer shape may be of any shape, for example polygonal such as triangular or quadrangular, as long as they have a structure capable of being sealed.

Here, the cover and container can be formed by a conventional injection molding method. As examples of materials, there are plastic materials such as polycarbonates (PC), polypropylenes (PP), polystyrenes and methylpentene copolymers (TPX) which excel in chemical resistance and heat resistance. In particular, methylpentene copolymers and polycarbonates which are highly transparent to light in the wavelength ranges used such as the ultraviolet, perceptible and infrared regions are considered to be suited to this application, but polycarbonates cost nearly four times the amount of polypropylenes. Although methylpentene copolymers are softer and more expensive than polycarbonates, their resin fluidity is good, thus offering the advantage of enabling thin molded articles to be made very easily by injection molding. Therefore, it is important to select materials in accordance with the required properties and cost.

While the wells formed on the surface of the container and the cover are joined by welding, each well must be sealed in a liquid-proof manner, so that the shape of the cover should preferable be flat and without curvature. If the cover is warped, portions will occur where contact is not achieved with the solution filling the wells when the cover is pushed against the wells, thus allowing air between the cover and the wells. As a result, there is the possibility of large amounts of air entering into the wells during welding. Therefore, the thickness of the cover must be such as to be thick enough to maintain flatness, but thin enough to retain thermal conductivity, that is, 0.15-3.0 mm, more preferably 0.25-1.5 mm.

On the other hand, if the front surface shape of the container is not flat as is the cover, it is not easy to weld it with the cover and seal of the liquid cannot be maintained, but the rear surface of the container need not be flat. However, by making the rear surface of the container, especially the rear surface portions directly underneath the wells flat as shown in Fig. 2, the microwell array can be positioned above a flat heat block in order to heat it. With the microtiter plates which are currently in general use, each well has a conical outer shape, so that the heat block must have conical holes in order to accommodate them. Additionally, if the hole portions of the heat block and the side walls of the conical microtiter plates do not make contact in a precise manner, it is not possible to uniformly and efficiently heat each well in a 384-well microtiter plate. Thus, by making the rear surface of the microtiter array flat, it is not necessary to use a heat block having a complicated shape and requiring a high degree of work precision, thereby allowing for heating on more economical devices such as hot plates. Furthermore, in the case of a microtiter plate, each well is usually sealed with a film coated with adhesive, but it is not possible to keep each well liquid-proof in a water bath with this type of sealing method using adhesives. However, since in the microwell array of the present invention, each well is sealed in a liquid-proof manner, it is possible to perform incubation (heating) in a water bath without using heaters or the like. Therefore, hundreds of microwell arrays can be simultaneously heated by using an isothermic bath.

Additionally, when the aqueous solution inside the wells of the microtiter plate are heated to a temperature at the level of boiling, it is no longer possible to sustain the seal with the sealing film affixed with adhesive, and the solution inside the well will evaporate unless held down from above the well by a mechanical force. Therefore, heaters such as thermal cyclers which are currently available are provided with lids for mechanically holding the film attached to the microtiter plate. However, since the microwell array of the present invention is sealed in a liquid-tight manner by means of ultrasonic welding with respect to each well, the seal can survive heating to temperatures where the solution inside the wells will boil, so that there is no risk of the solution inside the wells escaping outside the wells. That is, even if the microwell array is heated in a bath to 90-100 °C in order to modify the DNA, it is possible to maintain the seal on the wells. This kind of complete seal is possible because a portion of the cover and a portion of the container forming the microwell array are melted and fused by frictional heat. Therefore, the seal on each welded well, that is, the mechanical strength of the seal of each well is of the same level as the mechanical strength of the plastic which is the raw material of the microwell array itself. As a result, a seal which is incomparably stronger than the seals conventionally achieved by adhesives is achieved by ultrasonic welding. Thus, by using this microwell array, the solution inside the wells can be incubated (heat treatment) in a bath without using a heater or the like. Additionally, it is possible to simultaneously process thousands of microwell arrays by immersing them in an isothermic bath, obviating the need for thousands of heaters, and thereby allowing for inexpensive and speedy processing.

Additionally, if the rear surface of the container is flat, an object having the property of reflecting light can be placed along the rear of the container, so that as shown in Fig. 7, the light to be detected which is emitted from a fluorescent reagent in the well or the like can be reflected back to the detecting apparatus which is positioned above the container without allowing the light to escape to the rear of the container. Since the fluorescent light emitted from the reagent is usually emitted in all directions, the fluorescent light capable of being measured by the detecting apparatus can be roughly doubled by placing a reflective object at the rear surface, i.e. the S/N ratio can be doubled. On the other hand, if excitation light is entered from the rear side of the container and the photodetector is placed on the rear side, similar effects can be achieved by placing an object that reflects light above the cover. In general, reflecting materials comprising metals such as aluminum or stainless steel with polished surfaces or materials with metals of high reflectivity such as aluminum or gold coated on the surface of a solid have the property of reflecting light. Furthermore, by coating the rear surface of the container or the inner walls of the wells with a metal by means of vapor deposition or sputtering, it is possible to give them the property of reflecting light. Of course, the same effects can be achieved using commercially available mirrors as well.

Since the microwell array after welding the container and cover must be flat and have good thermal conductivity also, the thickness of the container and cover together should be 0.3-4.0 mm, preferably 0.5-3.0 mm. If the thickness of the container and cover together is less than 0.3 mm, the rigidity is too small, and it becomes difficult to perform welding of a 384-well array with uniform pressure.

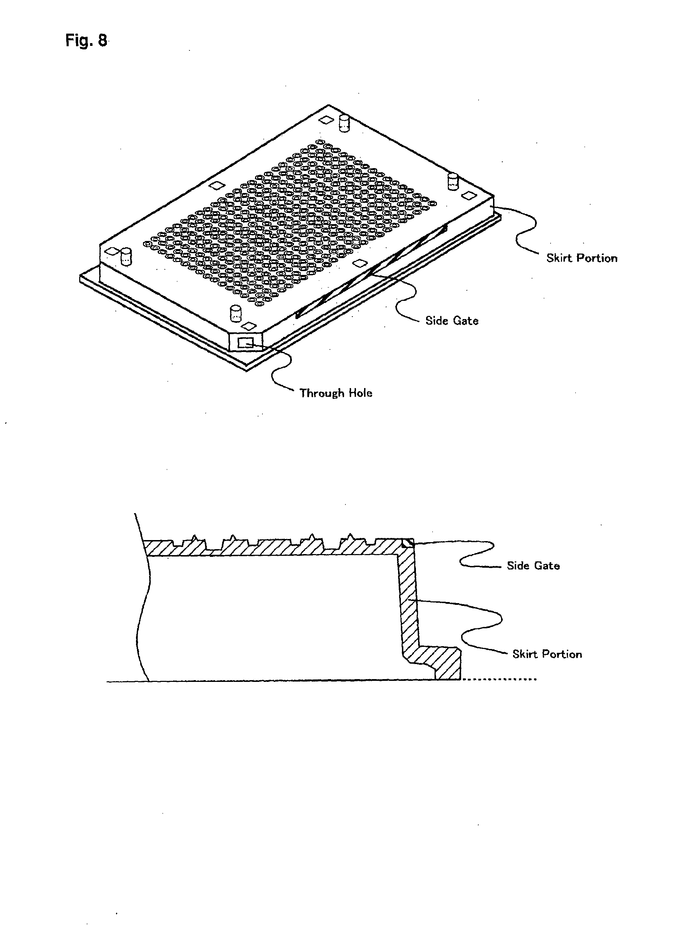

If a skirt portion is formed along the outer circumferential portion of the microwell array as shown in Fig. 8, it becomes less likely for the microwell array to warp, and the microwell array can be attached without using any special adapters when setting it in on the stage of a general-purpose distributive injection apparatus. Additionally, by providing through holes in the comers of the skirt portion, bubbles which have accumulated at the rear surface of the container are allowed to escape through the through holes during incubation in a water bath, so that the entire rear surface of the container can be heated uniformly.

Furthermore, when forming the container and cover by means of injection molding, it is possible to use a pin gate as is usual, but if the thickness is small as in the case of the present invention, the resin will not easily flow with a pin gate, thus largely warping the array. Since it will then become difficult to precisely spot the solution in each well when injecting minute amounts of solution with a syringe, it is desirable to pour the resin through a side gate as in Fig. 8.

Although the effects of leakage of light from adjacent wells, i.e. cross-talk cannot be ignored if the wells are provided at a high density, this can be prevented by mixing pigments or metallic micropowders into the raw material of the container to make the container non-transparent.

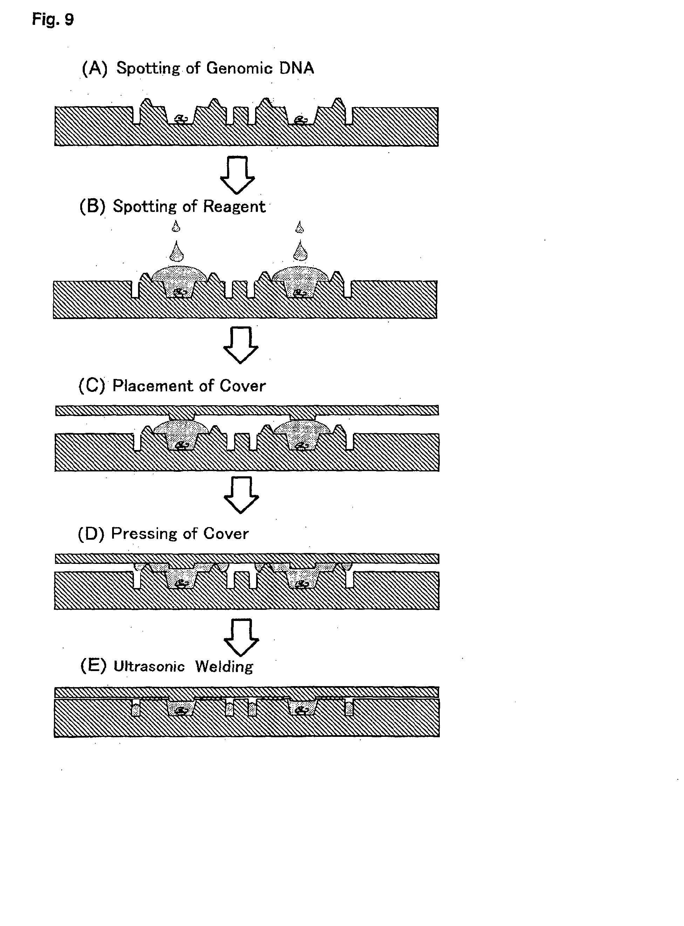

Fig. 9 shows the steps of spotting genomic DNA in the microwell array shown in Fig. 1, and after drying, injecting and sealing the reagent (liquid). The DNA referred to here is not DNA in their naturally occurring state as contained inside cells, but rather DNA which has been extracted from cells for reactions with enzymes, DNA dissolved directly in solution or DNA which has been amplified or chemically synthesized. In Fig. 9A, a minute amount of DNA has been spotted and the aqueous solution dried, and in Fig. 9B, the reagent for reacting with the DNA is spotted in an amount which is 30-90% more than the volume of the well. The liquid is affected by surface tension, so that the liquid in the portions which does not fit in the well bulges from the well and is held without being spilled. Next, by pushing the liquid filling the well outside the well by means of the expelling portions of the cover as shown in Figs. 9C and 9D, it is possible to seal the wells in such a manner that almost no air remains in the wells. Furthermore, by pressing the raised portions of the wells against the cover as in Fig. 9E and melting the raised portion by means of ultrasonic waves, the portions of contact between the raised portion and the cover can be joined, thus sealing the wells in a liquid-proof manner.

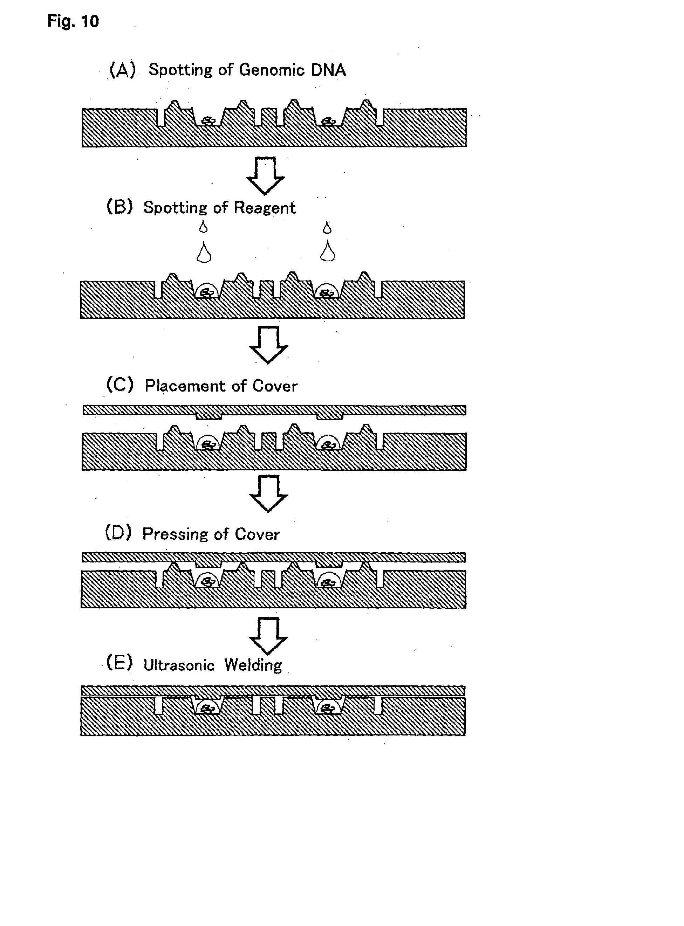

While it is desirable to provide 30-90% more liquid than the volume of the wells when spotting liquid in the wells, the reaction will progress even if there are a lot of air bubbles if the concentration of the liquid is extremely high because a large amount of fluorescent light will be emitted for detection, so that there will be cases where a reaction and detection can still be obtained by spotting fluid in an amount roughly equal to the volume of the wells after welding as in Fig. 10. Therefore, it is necessary to spot the liquid in an optimum amount in consideration of the sensitivity of the reaction and detection and the concentration of the reagent filling the wells.

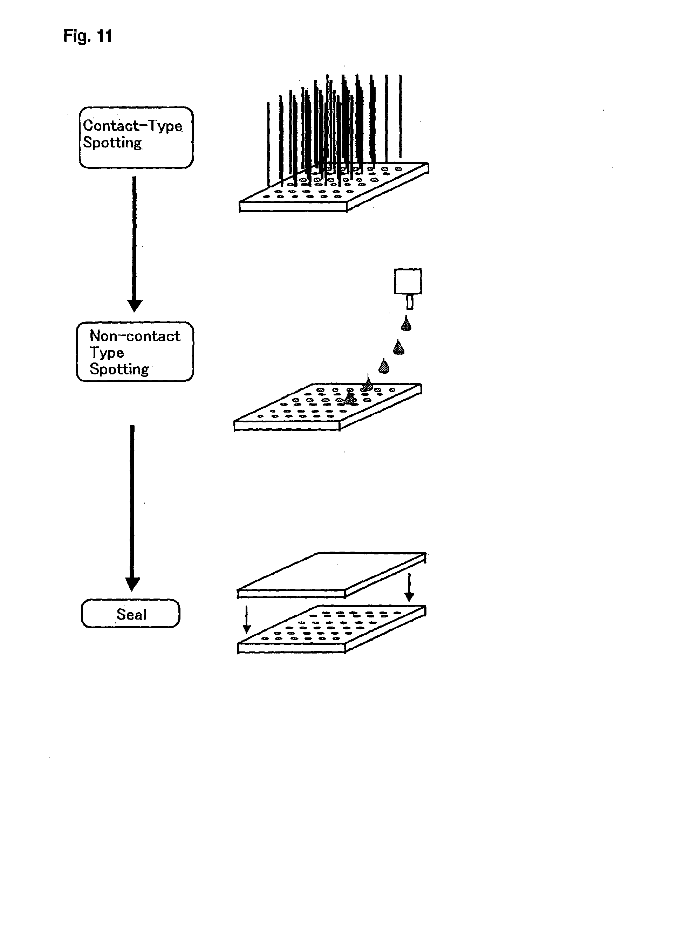

While it is possible to inject the solution by means of a contact-type injector since the container is first cleansed when solution is injected into each well, by using a contact-type injector when next injecting a different solution, the first solution and second solution will intermix, thus causing contamination, so that it is desirable to inject the solution by means of a non-contact type injector as shown in Fig. 11 in the second injection. Here, a contact-type injector refers to an apparatus for distributively injecting liquid to be spotted in wells by touching syringes or tips, or syringes or tips with drops of liquid adhering thereto when injecting the liquid into the microwell array. On the other hand, a non-contact type injector refers to an apparatus which can perform distributive injection without contacting the wells, by extruding the liquid by means of air pressure, a valve, a piezoelectric device or thermal expansion. Since in the microwell array of the present invention, there are cases in which the volume of the wells is small and the mouth portions are not very large, it is preferable to perform injection for the second and subsequent times by means of a non-contact type injector.

As another sealing method, it is possible to obtain a liquid-proof seal without using a non-contact type injector by spotting 384 types of liquid onto the cover surface by means of a contact-type injector and drying, then entering reagent into the 384 wells by means of a separate contact-type injector, and welding the cover and the container for each well. Additionally, in contrast thereto, it is possible to obtain a liquid-proof seal without using a non-contact type injector by spotting liquid onto 384 positions on the cover surface by means of a contact-type injector and drying, then entering different types of reagent in the 384 wells by means of a separate contact-type injector, and welding the cover and the container for each well.

Additionally, by drying the solution after injecting solution into the wells, it is possible to repeatedly inject different solutions into each well. DNA and fluorescent reagents do not greatly lose their properties even when dried, and enzymes can also often be dried. For this reason, reagents which are to be common ingredients in an assay can be pre-injected into the wells or the cover surface and dried, with the reagents which are different according to each well being added to the wells with a distributive injector. Furthermore, either the reagent or the sample or both can be distributively injected into the wells of the container, with the other part being held on the cover surface, after which the well and cover can then be joined by ultrasonic welding to induce a reaction between the reagent and sample in each well.

When welding together a cover and a container containing liquid, the ultrasonic waves must be directed to the welding portion. Ultrasonic welding is performed by converting ultrasonic electrical energy into mechanical vibrational energy, and applying pressure so as to generate a strong frictional heat at the surface of contact between the two parts to be welded, thereby melting the plastic and fusing them. The energy transmitted to the welding portion can generally be expressed as:

$$\text{Ultrasonic Energy} \quad \propto \quad (\text{force}) \times (\text{frequency of ultrasonic waves}) \times$$

$$(\text{amplitude of horn}) \times (\text{time required for welding})$$

$$\propto \quad (\text{output}) \times (\text{time required for welding})$$

As long as the right side of the above formula remains constant, it is possible to supply a constant amount of energy to the welding portion. Therefore, the energy transmitted to the welding portion can be held constant by prolonging the welding time or raising the horn amplitude even under conditions where the force must be set low. When the size of the parts to be welded is comparatively large and the overall length of the raised portion to be welded is large, there is an upper limit on the output of the ultrasonic apparatus, so that the energy is increased by prolonging-the normal welding time.

The ultrasonic waves for welding the container and cover are chosen from those which are sufficient for welding but simultaneously do not substantially damage DNA or proteins. Not substantially damaging DNA or proteins here signifies that enough DNA or proteins remain in the wells to enable subsequent analysis.

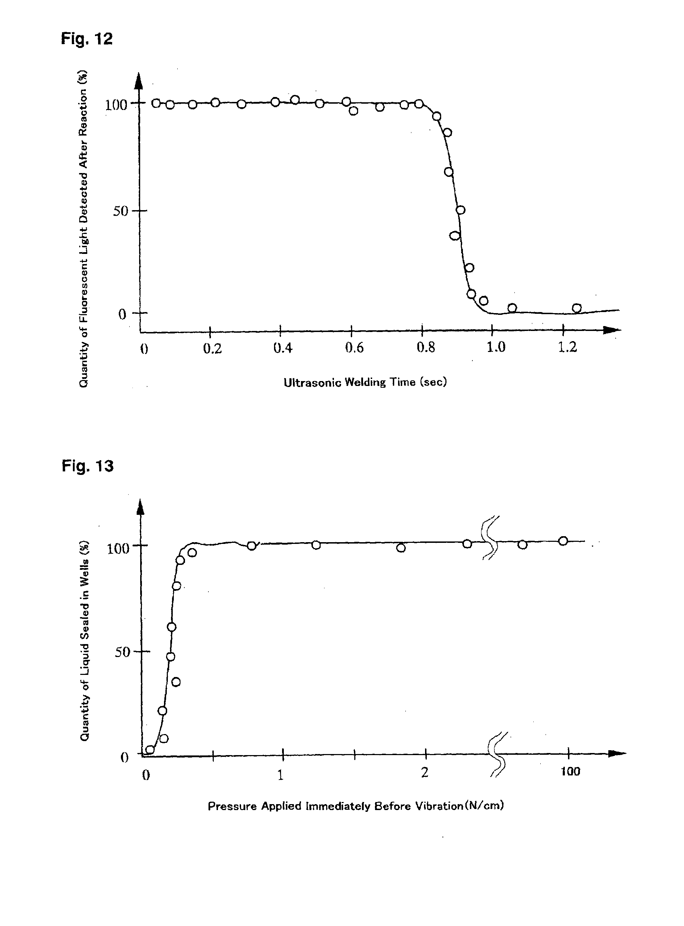

However, when there is liquid, proteins (enzymes), DNA and the like in the container which is to be joined by welding as in the present invention, the DNA or proteins may be severed or lose their activity due to high temperatures if put under ultrasonic vibrations for a long time, thus not allowing the chemical reaction in the wells to progress. Additionally, if a liquid is left under vibrations for a long time, it can fly off from the wells. Fig. 12 shows the experimental results for a case where an invader reagent (enzyme, fluorescence, etc.) and DNA have been put in the wells of a microwell array formed from a TPX (methylpentene copolymer), the wells were sealed in a liquid-proof manner by means of ultrasonic waves, and the amount of fluorescent light detected after the reaction was plotted with respect to welding time. At this time, the force was approximately 40 N per cm of the welding portion, the frequency of the ultrasonic waves was 20 kHz, and the amplitude of the horns was 36 microns. When the welding time was within the range of 0.05-08 seconds, the enzyme was active, and the fluorescent molecules cleaved as a result of the enzyme reaction were detected as shown in Fig. 12. However, when the welding time exceeded 0.8 seconds, the amount of fluorescent light detected decreased, with absolutely no fluorescence being detected when welding for 1 second. These results demonstrate that the sealing of DNA and proteins by ultrasonic waves must be done within 0.8 seconds.

Fig. 13 shows the relationship between the amount of fluid remaining in the well and the force (vibration pressure) applied to the cover at the time the vibration of the horn is begun, when an invader reagent (such as an enzyme) and DNA have been put into the wells of a microwell array composed of TPX (methylpentene copolymer) and the wells are being sealed for liquid-proofness by means of ultrasonic waves. At this time, the frequency of the ultrasonic waves was 20 kHz, the amplitude of the horn was 36 microns and the welding time was 0.25 seconds. As shown in Fig. 13, the wells can be sealed with almost no bubbles if the horn vibrations are started at the point where a force of 0.3-100 N, more preferable 30-100 N is applied per centimeter of length of the raised portion being fused. Here, the "length of the raised portion" indicates the length of the portion to be fused formed around the wells. For example, the sum of the lengths of raised portions having a diameter of 0.19 cm in a 384-hole microwell array will be:

$$0.19 \times \pi \times 384 = 229 \text{ cm}$$

Therefore, if it is assumed that a force of 5000 N is applied to the entire microwell array when starting the ultrasonic vibrations, then by:

$$5000 \div 229 = 22 \text{ N/cm}$$

a force of 22 N can be considered to have been applied to each cm of length of raised portion to be fused. If the vibrations are begun with a pressure of less than 0.3 N, liquid in the wells are thrown out of the wells by the vibrations, thus making it difficult to seal the wells without intermixture of air bubbles in the liquid even if the welding time is shortened or the horn amplitude is made smaller. By applying a force to the entire cover prior to the vibrations from the horn, i.e. by means of pressure vibration, a sufficiently tight contact can be achieved between the cover and raised portions around the wells, thus enabling liquid to be sealed in the wells without allowing any outitow.

Normally, in ultrasonic welding of plastic parts composed of TPX or PC, a horn amplitude of respectively 45 microns and 60 microns is held to be necessary, but when liquid is contained in the wells as in the present invention, the amplitude of the horn should be set to 40 microns or less in order to prevent liquid from splashing out of the wells during the weld. On the other hand, if the amplitude is less than a lower limit amplitude of 30 microns, the energy of the ultrasonic vibrations will not be efficiently transmitted to the portion being welded, so that the frictional heat is insufficient for welding, resulting in a bad weld. Thus, since the pressure applied during the vibrations should preferably be 30-100 N/cm per unit length of the portion being welded, it should in this particular example be:

$$229 \text{ cm} \times 30\text{-}100 \text{ N/cm} = 6870\text{-}22900 \text{ N}$$

That is, it is preferable to have equipment which is capable of applying a force of 7000-23000 N at maximum output.

DNA were distributively injected and dried in a microwell array having 384 wells as described above, after which 0.4 µl (well capacity 0.6 µl) of reagent were added, the result covered, and the wells welded by ultrasound. At this time, when the horn amplitude was set to 36 microns, the welding time to 0.25 seconds and the applied pressure to 10000 N, the maximum oscillation output at welding was 4.1-5.0 kW. The reagent in the wells was not thrown out of the wells during the weld, and the hold and seal at the peripheral portions of the wells after the ultrasonic weld were good.

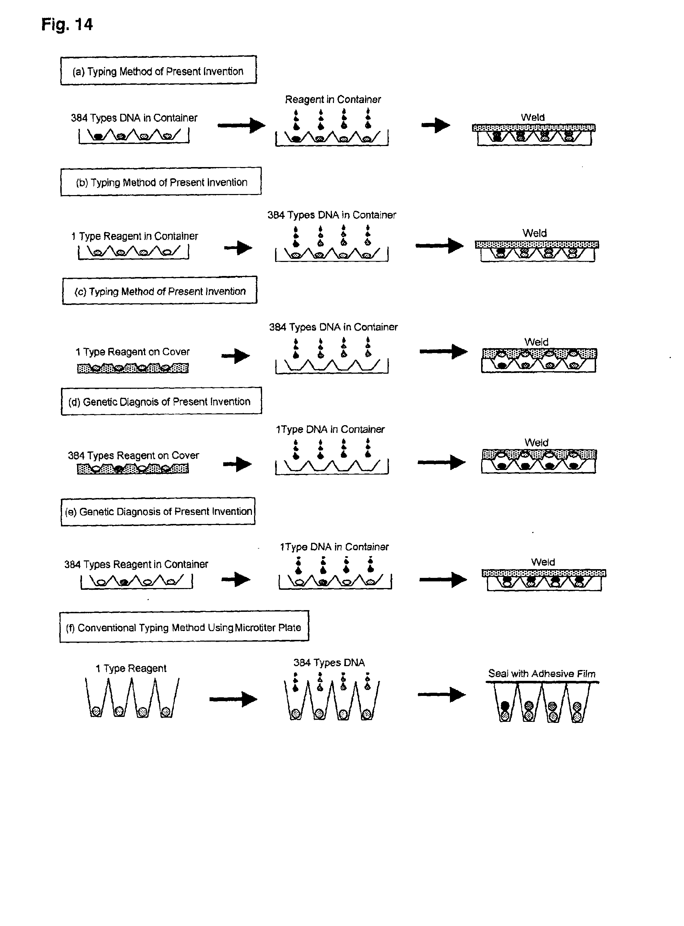

When performing single nucleotide polymorphism (SNP) typing by means of the Invader method orTaqMan PCR method using the above-described microwell array, distributive injector and ultrasonic welding apparatus, the procedure must as a system follow the order shown in Fig. 14(a).

(1) Each microwell is labeled with a bar code corresponding to information concerning the sample and reagent which are injected therein. Bar codes are also provided on the mother plate holding the 384 types of DNA (samples, i.e. the DNA of 384 people).

(2) The 384 types of DNA to be analyzed are distributively injected in suitable quantities into the respective wells on the container surface of the microwell array by a contact-type distributive injector, then the moisture is vaporized to dry. At this time, the numbers corresponding to the samples in the bar codes provided on the microwell array and the sample numbers of the mother plate are made to match. Additionally, after the injections are completed, the server controlling the procedure is sent information to the effect that the first injection procedure for the microwell array provided with the bar codes has been completed.

(3) A reagent common to all of the wells on a container surface (differing by the microwell array) is distributively injected by means of a non-contact-type injector. The types of reagent used at this time are made to match with the bar code numbers corresponding to the reagent provided on the microwell array. After injection, the bar codes of the microwell arrays for which injection has been completed are read by a bar code reader, and the server controlling the procedure is sent information to the effect that the second injection procedure for the microwell array provided with the bar codes has been completed.

(4) Immediately after injection, before the reagent in the wells dries, the cover is pressed onto the container, and the wells welded to the cover by ultrasound.

(5) The microwell array is set in an isothermic bath or a heating device such as a thermal cycler, and the reagent and samples (DNA) allowed to react (incubated) for a standard period of time.

(6) After the reaction, the microwell array is set in a fluorescent evaluation device (plate reader), the bar codes on the microwell array are read by a bar code reader, and the intensity of the fluorescent light is read for each well. After measurement, the server controlling the procedure is sent information to the effect that the fluorescent analysis procedure for the microwell array provided with the bar codes has been completed.

(7) By making use of the fact that certain base sequences correspond to certain fluorescent colors, the SNP sequence information of the 384 types of DNA are analyzed on the basis of the color and intensity of the fluorescent light detected, so as to analyze the SNP frequency (typing).

The above-described bar code numbers can, for example, be written as follows:

aaa-bbb

where aaa is a number indicating the type of reagent which is injected into the microwell array and bbb is a number indicating the type ofDNA injected into the microwell array.

The SNP frequency analysis method described above is for the case where the DNA and reagent are sequentially injected into the wells, mixed together, then allowed to react. However, as an alternative method, it is possible to inject and dry the reagent (or DNA) on the cover surface with a contact-type distributive injector, inject an aqueous solution (or reagent) containing DNA into the wells with a contact-type injector, and press the cover onto the container prior to the moisture in the wells evaporating and drying to mix and react the reagent on the cover surface and the DNA in the wells (Fig. 14(c)). There is also the method shown in Fig. 14(b).

While 384 types of DNA are injected into respective wells and a common reagent injected into all 384 wells in the above-described SNP frequency analysis (typing) method, if a microwell array is used for genetic diagnosis, then it is possible to pre-inject 384 types of reagent into the 384 wells, dry them, and inject a single person's DNA into all 384 wells, thereby enabling 384 types of SNP information of a single person, i.e. 384 types of genetic information to be obtained (Fig. 14(e)).

Thus, the flow of procedures for the case of performing genetic diagnosis is specifically as follows.

(1) Each microwell is labeled with a bar code corresponding to information concerning the sample and reagent which are injected therein. Bar codes are also provided on the mother plate holding the 384 types of reagent (reagents corresponding to 384 types of genes).

(2) The 384 types of reagent to be analyzed are distributively injected in suitable quantities into the respective wells on the container surface of the microwell array by a contact-type distributive injector, then the moisture is vaporized to dry. At this time, the numbers corresponding to the reagents in the bar codes provided on the microwell array and the reagent numbers of the mother plate are made to match. Additionally, after the injections are completed, the server controlling the procedure is sent information to the effect that the first injection procedure for the microwell array provided with the bar codes has been completed.

(3) A single person's DNA are injected into all wells on the container by means of a non-contact-type injector. The DNA (that of a single person) used at this time and the bar code number corresponding to the DNA (that of a single person) provided on the microwell array are made to match. After injection, the bar codes of the microwell arrays for which injection has been completed are read by a bar code reader, and the server controlling the procedure is sent information to the effect that the second injection procedure for the microwell array provided with the bar codes has been completed.

(4) Immediately after injection, before the mixed solution in the wells dries, the cover is pressed onto the container, and the wells welded to the cover by ultrasound.

(5) The microwell array is set in an isothermic bath or a heating device such as a thermal cycler, and the reagent and samples (DNA) allowed to react (incubated) for a standard period of time.

(6) After the reaction, the microwell array is set in a fluorescent evaluation device (plate reader), the bar codes on the microwell array are read by a bar code reader, and the intensity of the fluorescent light is read for each well. After measurement, the server controlling the procedure is sent information to the effect that the fluorescent analysis procedure for the microwell array provided with the bar codes has been completed.

(7) By making use of the fact that certain base sequences correspond to certain fluorescent colors, 384 SNP (gene) types are analyzed for a single person to perform the diagnosis. Aside from the above-described method, it is possible to perform genetic diagnosis by means of the method shown in Fig. 14(d).

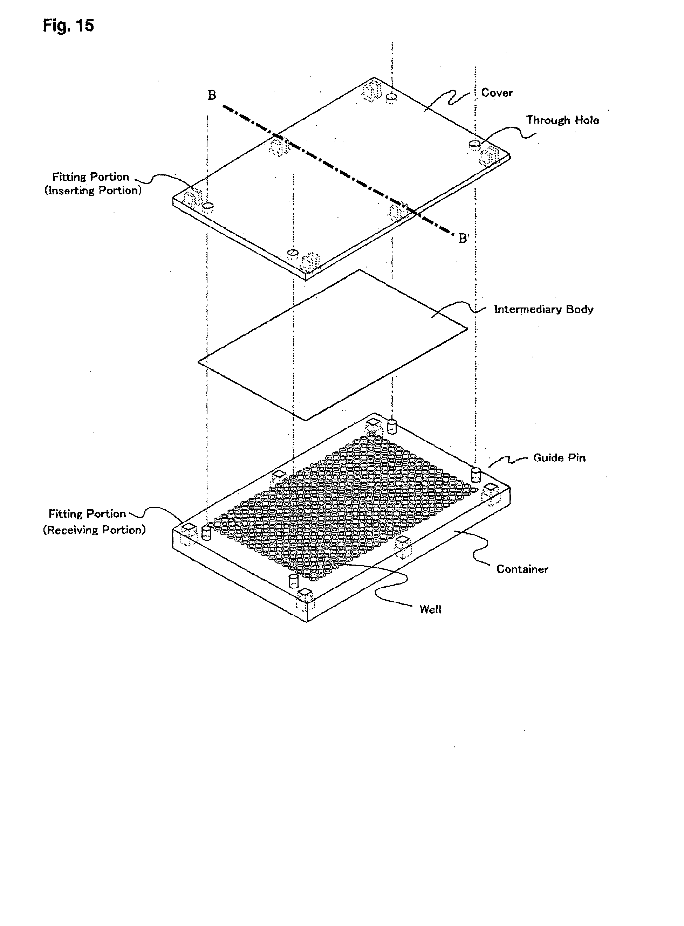

Fig. 15 is a perspective view of a different microwell array according to the present invention. This one also has 384 wells formed on the surface of a plastic container with a lateral spacing of 4.5 mm, such that the liquid loaded in the wells can be sealed by pressing an intermediary body against the container from above. In order to reliably align the positions of the cover and the container to ease their joining, through holes, guide pins and fitting portions are provided at the four comers as in Fig. 1. The intermediary body refers to a material for sealing composed of a film, sheet, adhesive or bond, and more specific examples include the combinations "adhesive + sheet + adhesive", "adhesive + film + adhesive", "bond + sheet + adhesive", "bond + film + adhesive", "bond + sheet + bond", "bond + film + bond", "adhesive + sheet", "bond + film", "sheet only", "adhesive only" and "bond only". When the cover alone, being flat and formed of a rigid material, is not sufficient to form a seal, an intermediary body composed of a substance having flexibility such as a synthetic resin is used to allow a seal to be formed. Then, the tightness of the seal can be increased by using an adhesive or a bond. Here, a sheet is a plastic that has been rolled to a thickness of at least 0.10 mm, and a film is a plastic that has been rolled to a thickness of less than 0.10 mm.

By combining intermediary bodies such as the above, the productivity can be improved. For example, when performing the sealing work, both sides of a sheet are pre-coated with adhesive, then lubricant paper on one surface of the sheet is peeled off and the sheet is adhered to the cover, thereby uniting the cover and sheet. Then, the lubricant paper on the other side of the sheet is peeled off, and the united cover and sheet are pressed against the wells formed on the container surface. In this way, it is possible to seal the wells in a liquid-proof manner.

As another method, it is possible to pre-coat the surface of the cover with adhesive, then press the cover which has been united with the adhesive against the wells on the surface of the container. Additionally, it is of course possible to use, for example, a sheet having elasticity as the intermediary body.

As the material of the sheet, it is preferable to use polyolefins, polyethylenes, silicone rubber, polyurethane rubber, elastomers or the like which have elasticity and are capable of sealing liquid inside the cells. Additionally, as the form thereof, it is particularly preferable to have a foamed sheet which is locally compressed at the portions contacting the raised portions of the wells capable of maintaining a tight seal.

The thickness of the intermediary body should preferably be 0.1-1.5 mm, more preferably 0.3-1.0 mm because if too thin, the liquid will not be able to be sealed and if too thick, the thermal conductivity will decrease.

Here as well, the shape of the cover should preferably be flat and without curvature. If the cover is warped, parts of the surface of the intermediary body will not contact the wells when the intermediary body is pressed against the wells by means of the cover, and as a result, air will reside between the intermediary body and the wells. Therefore, there is a possibility of air mixing into the wells. Accordingly, the thickness of the cover should be 0.15-3.0 mm, more preferably 0.25-1.5 mm, which has the minimum thickness required to maintain flatness but still allows for a certain degree of thermal conductivity.

When the amount of fluorescent light detected is small, it is possible to increase the amount of detectable fluorescent light by using a film or sheet which reflects light as the material composing the intermediary body. Additionally, the rate of reflection can be increased by coating the surface of a film, sheet or cover with a reflective metallic film of gold, aluminum or the like by means of vacuum deposition or sputtering. In this case, the excitation light and fluorescent light are both respectively emitted and detected from the reverse side of the container.

As another method, it is possible to coat the inner walls of the wells with a light-reflecting metallic film, use a transparent intermediary body, and emit the excitation light from the cover side. In this way, it is possible to offer the optimum conditions for a desired assay by controlling the properties of the intermediary body such as its thermal conductivity and optical properties.

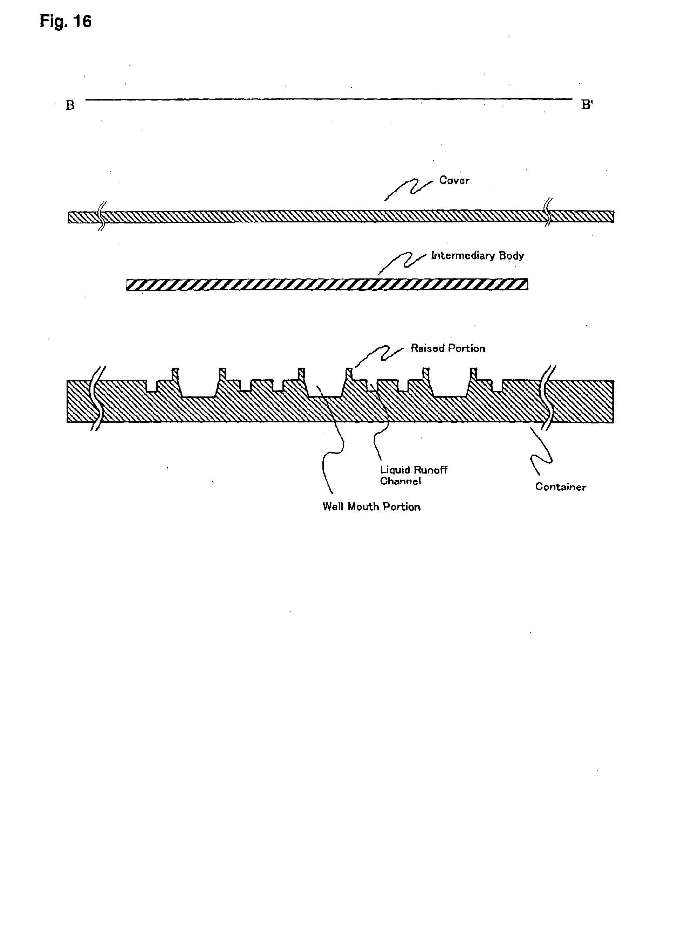

Fig. 16 is a section view along the line B-B' of Fig. 15, and as shown in the drawing, a well is composed of the mouth portion of the well, a raised portion and a liquid runoff channel. In this example, the intermediary body is a sheet having elasticity, and by applying downward pressure to the cover, it is possible to press the sheet against the raised portion and seal the liquid in the wells by means of the sheet. In order to affix the cover to the container with the sheet in between, a fitting portion as shown in Fig. 15 is formed, so that by fitting the insertion portion formed in the cover into the receiving portion formed in the container, they can be efficiently and conveniently locked.

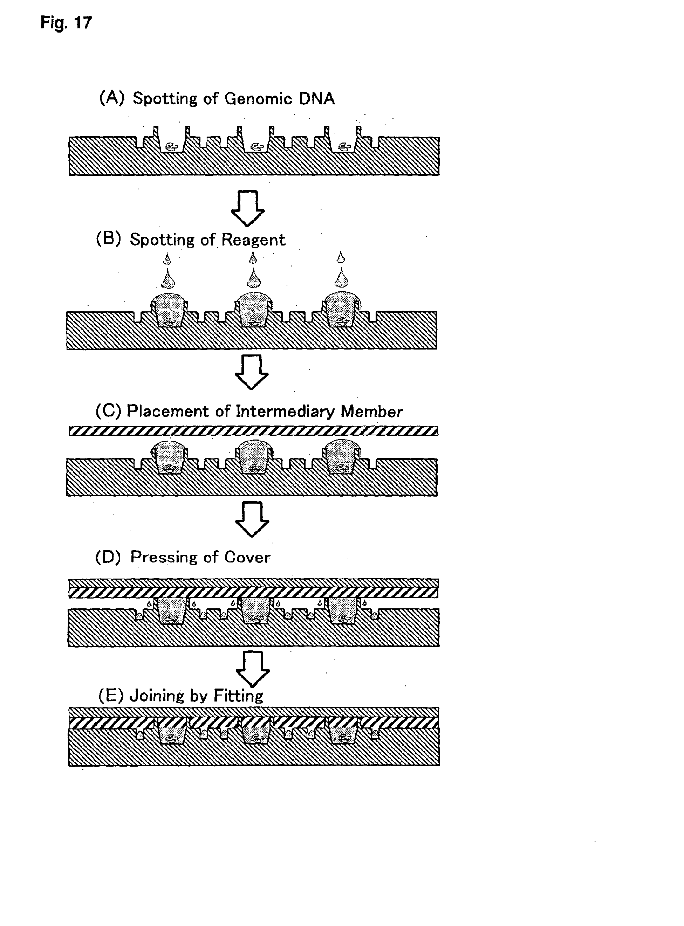

Fig. 17 shows the steps for spotting the genomic DNA in the microwell arrays shown in Fig. 15, drying, then injecting and sealing the reagent (liquid). In Fig. 17A, a minute quantity of DNA is spotted, and in Fig. 17B, the reagent to be reacted with the DNA is spotted in an amount 30-90% greater than the volume of the wells. The liquid has surface tension, so that the liquid which does not fit in the well is held in a bulge so as not to spill from the well. Next, by pressing the liquid filling the wells out of the wells by means of the intermediary body as shown in Figs. 17C and 17D, the wells can be sealed without any air remaining.

In the present invention as shown in Fig. 15, if the intermediary body is non-transparent, it is not possible to shine light onto the solution in the wells from above the wells, but by turning the container upside down, the light (excitation light or the like) can be illuminated onto the wells without being blocked by the intermediary body. Additionally, the fluorescent light can be detected from the same direction as that from which the light was shined.

In the present invention as shown in Fig. 15, the ultrasonic energy is not directly transmitted to the liquid inside the wells when the liquid is sealed in the wells, so that this example is particularly suited to cases where the liquid inside the wells should not be exposed to the plastic welding temperature (200-250 °C) even temporarily.

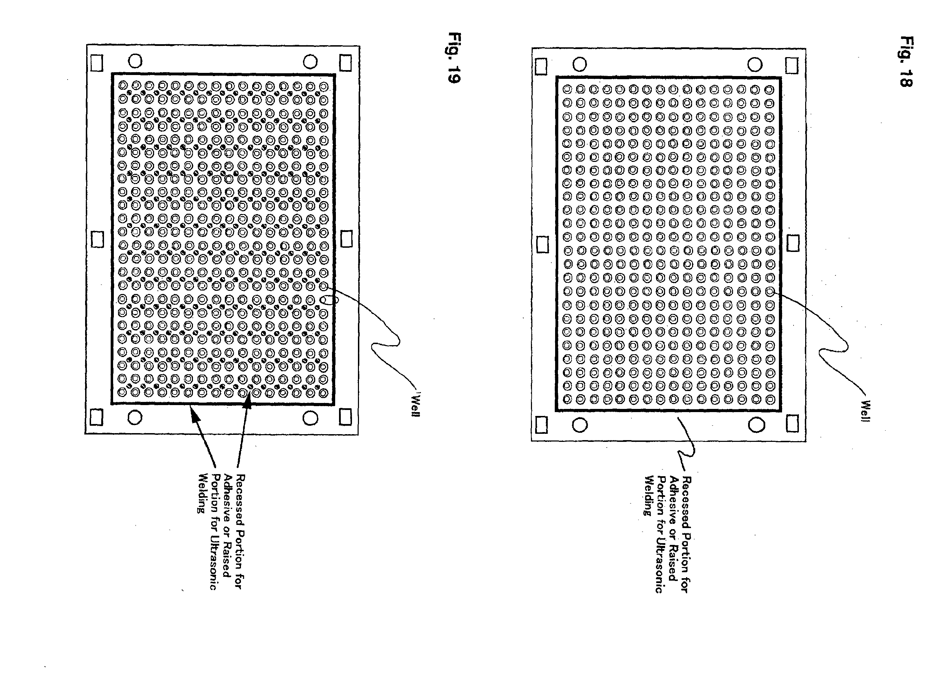

Additionally, if it is desired to firmly join the cover and the container and make the well and intermediary body liquid-proof, it is possible to form elongate channels (recessed portions) for holding adhesive at the peripheral portions of the container as shown in Fig. 18. By pouring adhesive therein, adhesion can be achieved by fitting protruding portions formed on the cover into the recessed portions. Alternatively, the periphery can be surrounded by a raised portion and ultrasonically welded to form the seal.

Since the standard size of a microwell array is relatively large with a width of roughly 8 cm and a length of roughly 12 cm, there may be cases in which it is difficult to apply a uniform pressure on the entire intermediary body covering the wells, as a result of which there will be some variance in the seal on the wells. In this case, it is possible to provide channels (recessed portions) for holding adhesive at positions surrounded by four wells such as shown in Fig. 19, so that by pouring adhesive therein and fitting protruding portions formed on the cover into the channels of the container, it is possible to maintain a uniform tightness of seal.

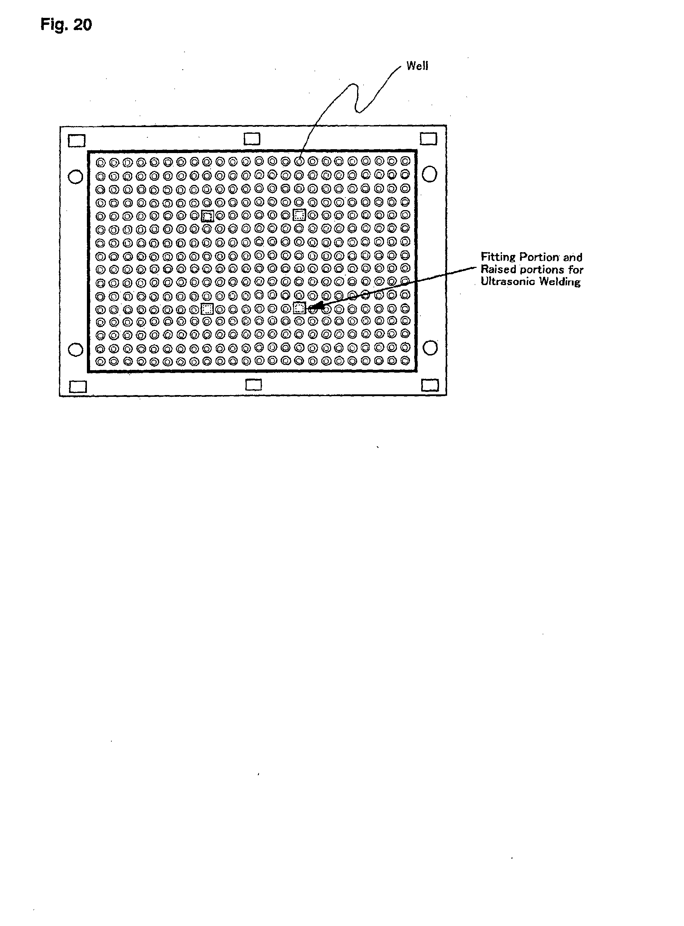

Additionally, as another example, the pressure of the cover can sometimes be difficult to apply to the intermediary body covering the wells positioned in the central portion of the microwell array, thus degrading the seal. In this case, the intermediary body can be prevented from separating from the wells by providing a fitting portion and surrounding raised portions at the central portion of the microwell array as shown in Fig. 20.

In yet another example, the intermediary body can be formed by pre-coating the surface of a sheet or film with adhesive or bond, and when covering the wells with the intermediary body, adhering the intermediary body to the container to seal the wells in a liquid-proof manner. In this case, the thickness of the coated materials should also be included in the thickness of the intermediary body. Accordingly, when considering the tightness of the seal and the thermal conductivity, the thickness of the sheet or film together with the coated materials should be 0.15-3.0 mm, more preferably 0.25-1.5 mm.

Thus, the expression "intermediary body" includes all sheets, films, adhesives and bonds which are sandwiched between the cover ad the container, and the "thickness of the intermediary body" corresponds to the thickness which is the sum of all such materials from the viewpoint of tightness of seal and thermal conductivity.

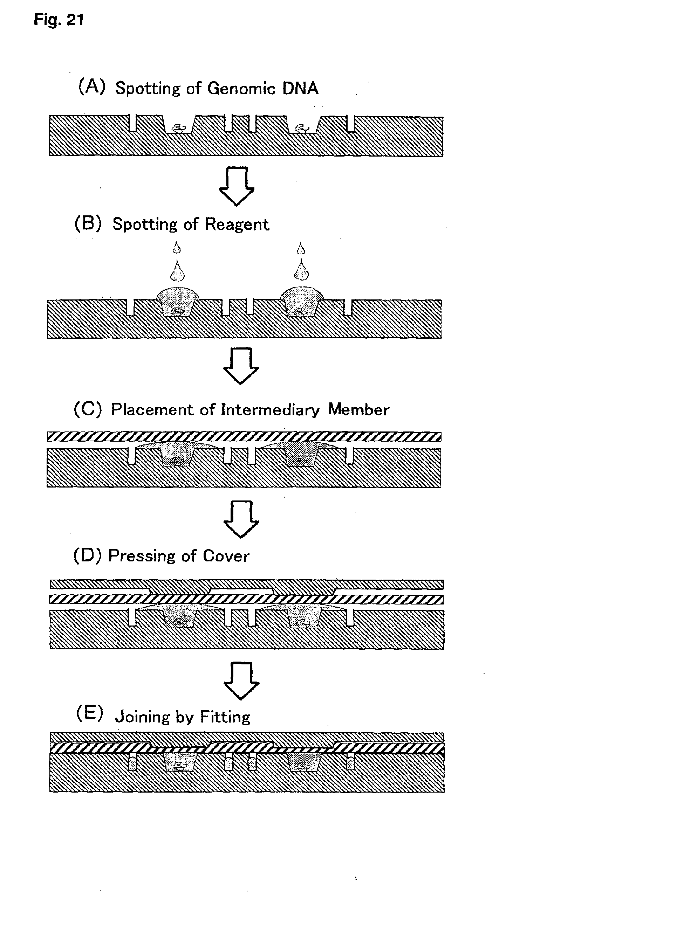

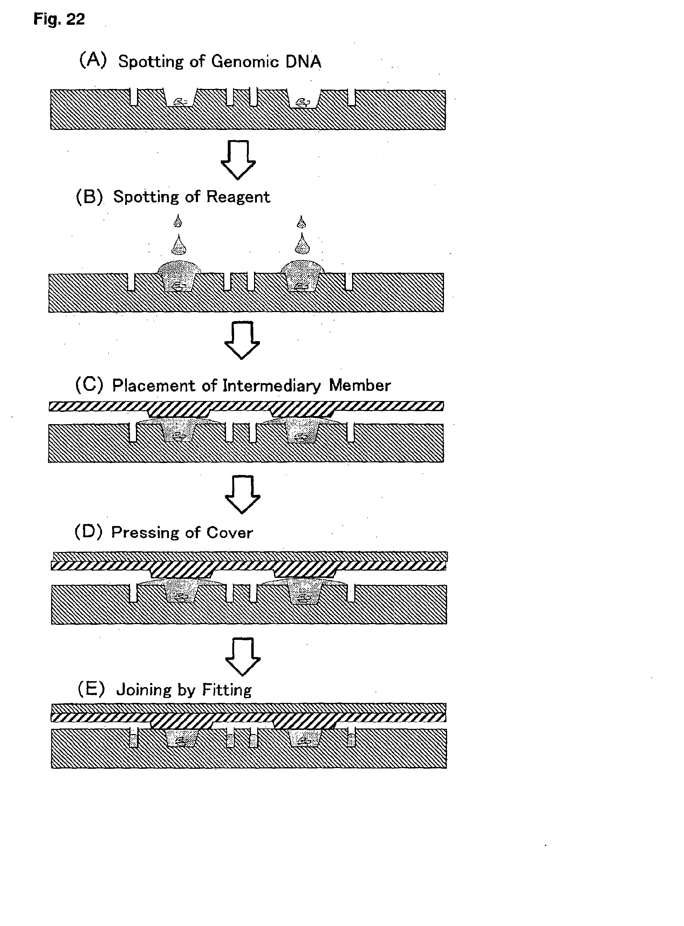

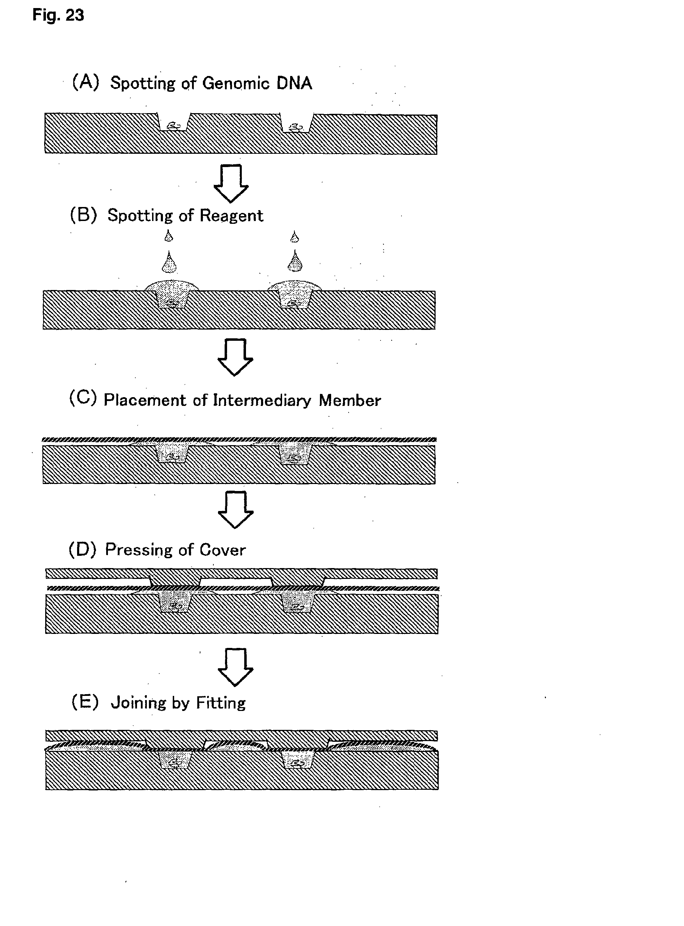

Aside from the above-described methods shown in Figs. 1 and 15, the liquid can be sealed inside the wells by the methods shown, for example, in Figs. 21-24. In Fig. 21, instead of providing raised portions in the container, a liquid expelling portion is formed in the cover, so that the solution filling the wells can be pushed out to the liquid runoff channels to obtain a seal without any air bubbles in the well. In Fig. 221, the liquid expelling potions are formed on the intermediary body, so that by pushing the intermediary body onto the wells, the extra solution which bulges out of the wells can be pushed out to the runoff channels so as not to leave air bubbles in the wells. In Fig. 23, no liquid runoff channels are provided, but the extra liquid is pushed out of the wells by means of expelling portions formed in the cover, thus preventing air bubbles from mixing into the wells. Additionally, in Fig. 24, liquid expelling potions and raised portions for ultrasonic welding are provided on the cover side, thereby allowing for a tight seal without any air bubbles residing in the wells.

Examples and Comparative Examples

Herebelow, examples and comparative examples of the present invention shall be given to give a more detailed description.

Examples 1-9

Experiment

A cover and container were produced by forming a mold, and injection molding methylpentene copolymers and polycarbonates in the mold. The size of the cover was 81 mm $\times$ 123 mm $\times$ 0.4 mm, and the size of the container was 81 mm $\times$ 123 mm $\times$ 1.6 mm. 384 wells with a trapezoidal cross section were formed on the surface of the container, their size being such that the diameter of the mouth portion was 1.3 mm, the diameter of the bottom portion was 1.1 mm and the depth was 0.8 mm (volume 0.9 $\mu$L/well). The height of the raised portions was 0.4 mm, with an inner diameter of 1.4 mm and outer diameter of 2.4 mm, and the runoff channels had an inner diameter of 3.0 mm, an outer diameter of 4.0 mm and a depth of 0.6 mm. The height of the liquid expelling portions formed on the cover was 0.2 mm, with an outer diameter of 0.9 mm.

First, bar codes corresponding to the samples and reagents were attached to the microwell array, and bar codes were also attached to a mother plate holding 384 types of DNA (the DNA of 384 people). Then, 1 $\mu$L, or 0.2 $\mu$L of each of the 384 different types of genomic DNA (10 ng/$\mu$L, 20 ng/$\mu$L, 40 ng/$\mu$L) were injected by means of a spotting apparatus into the respective wells of the container placed on an experimental stand. After leaving the container in the atmosphere and allowing the solvent to evaporate, roughly 1.6 $\mu$L, or 0.2 $\mu$L of a reagent for the Invader process having a fluorescence intensity peak at a wavelength of 570 nm was injected into each well by means of the non-contact type spotting apparatus. After injecting the reagent, the cover was pressed against the wells so as to expel the extra reagent bulging out from the wells, and the liquid in the wells was sealed. Then, the raised portions at the mouth portions of the wells were welded to the cover by means of an ultrasonic apparatus. An ultrasonic welding apparatus wherein the frequency of the ultrasonic waves was 20 kHz, the amplitude of the horns was 36 microns, and the maximum oscillation output was 5.0 kW was used. In a liquid-proof state, the DNA was denatured at 95 °C for 5 minutes, after which it was allowed to react in an isothermic bath of 63 °C for 4 hours, and after the reaction, the fluorescent light intensity was measured by a plate reader for SNP frequency analysis (typing). Upon completion of each procedure, the bar codes were read by the bar code reader, and the state of progress in the procedure for each microwell array was controlled by a computer functioning as a server. The tightness of the seals on the wells was good, with no air bubbles being apparent, neither immediately after welding nor after reacting for four hours. The frequency of the ultrasonic waves was held at 20 kHz and the amplitude of the horns at 36 microns, while the quantity of genomic DNA, welding time and vibration pressure were changed as shown in Table 1. Additionally, in Example 8, a mirror for reflecting light was positioned behind the container. The results are shown in Table 1.

Examples 10-18

Experiment

A cover and container were produced by forming a mold, and injection molding methylpentene copolymers and polycarbonates in the mold. The size of the cover was 81 mm × 123 mm × 0.4 mm, and the size of the container was 81 mm × 123 mm × 1.6 mm. 384 wells with a trapezoidal cross section were formed on the surface of the container, their size being of two types, those wherein the diameter of the mouth portion was 1.6 mm, the diameter of the bottom portion was 1.4 mm and the depth was 0.6 mm (volume 1.1 μL/well), and those wherein the diameter of the mouth portion was 1.6 mm, the diameter of the bottom portion was 1.4 mm and the depth was 0.8 mm (volume 1.4 μL/well). The height of the raised portions was 0.5 mm, with an inner diameter of 1.6 mm and outer diameter of 2.0 mm, and the runoff channels had an inner diameter of 2.5 mm, an outer diameter of 3.1 mm and a depth of 0.6 mm. As an intermediary body provided between the cover and the container, a foamed sheet of polyolefin (0.5 mm thick) was used.

First, bar codes corresponding to the samples and reagents were attached to the microwell array, and bar codes were also attached to a mother plate holding 384 types of DNA (the DNA of 384 people). Then, 1 μL of 384 different types of genomic DNA (10 ng/μL, 20 ng/μL, 40 ng/μL) was injected by means of a spotting apparatus into the respective wells of the container placed on an experimental stand. After leaving the container in the atmosphere and allowing the solvent to evaporate, roughly 1.6 μL and 2.0 μL of a reagent for the Invader process having a fluorescence intensity peak at a wavelength of 570 nm was injected into each well by means of the spotting apparatus. After injecting the reagent, the sheet and cover were pressed sequentially against the wells so as to expel the extra reagent bulging out from the wells, and the liquid in the wells was sealed. Then, the raised portions at the mouth portions of the wells were welded by means of an ultrasonic apparatus to form a liquid-proof seal, under which the DNA was denatured at 95 °C for 5 minutes, after which it was allowed to react in an isothermic bath of 63 °C for 4 hours, and after the reaction, the fluorescent light intensity was measured by a plate reader for SNP frequency analysis (typing). Upon completion of each procedure, the bar codes were read by the bar code reader, and the state of progress in the procedure for each microwell array was controlled by a computer functioning as a server. At this time, the container was flipped upside-down, and illuminated with light from the side of the wells not blocked by the sheet, and the fluorescent light was detected from the same side. The tightness of the seals on the wells was good, with no air bubbles being apparent, neither immediately after welding nor after reacting for four hours. The frequency of the ultrasonic waves was held at 20 kHz and the amplitude of the horns at 36 microns, while the quantity of genomic DNA, welding time and vibration pressure were changed as shown in Table 1.

Example 19

Experiment

A cover and container were produced by forming a mold, and injection molding methylpentene copolymers and polycarbonates in the mold. The size of the cover was 81 mm × 123 mm × 0.4 mm, and the size of the container was 81 mm × 123 mm × 1.6 mm. 384 wells with a trapezoidal cross section were formed on the surface of the container, their size being such that the diameter of the mouth portion was 1.3 mm, the diameter of the bottom portion was 1.1 mm and the depth was 0.8 mm (volume 0.9 μL/well). The height of the raised portions was 0.4 mm, with an inner diameter of 1.4 mm and outer diameter of 2.4 mm, and the runoff channels had an inner diameter of 3.0 mm, an outer diameter of 4.0 mm and a depth of 0.6 mm. The height of the liquid expelling portions formed on the cover was 0.2 mm, with an outer diameter of 0.9 mm.

First, bar codes corresponding to the samples and reagents were attached to the microwell array, and bar codes were also attached to a mother plate holding 384 types of DNA (the DNA of 384 people). Then, 1 μL of 384 different types of genomic DNA (10 ng/μL) was injected by means of a spotting apparatus into the respective wells of the container placed on an experimental stand. After leaving the container in the atmosphere and allowing the solvent to evaporate, roughly 1.6 μL of a reagent for the TaqMan process having a fluorescence intensity peak at a wavelength of 570 nm was injected into each well by means of the spotting apparatus. After injecting the reagent, the cover was pressed against the wells so as to expel the extra reagent bulging out from the wells, and the liquid in the wells was sealed.

Then, the raised portions at the mouth portions of the wells were welded to the cover by means of an ultrasonic apparatus to make them liquid-proof. The frequency of the ultrasonic waves was 20 kHz, and the amplitude of the horns was 36 microns. After denaturing the DNA for 10 minutes at 95 °C, a cycle of incubation of 1 minute at 95 °C and 3 minutes at 60°C was repeated 40 times in a thermal cycler. After the reaction, the fluorescent light intensity was measured by a plate reader for SNP frequency analysis (typing). Upon completion of each procedure, the bar codes were read by the bar code reader, and the state of progress in the procedure for each microwell array was controlled by a computer functioning as a server. The tightness of the seals on the wells was good, with no air bubbles being apparent, neither immediately after welding nor after the reaction. The frequency of the ultrasonic waves was held at 20 kHz and the amplitude of the horns at 36 microns, while the quantity of genomic DNA, welding time and vibration pressure were changed as shown in Table 1. The results are shown in Table 1.

Comparative Examples 1-3

Experiment

A cover and container were produced by making a mold, then injection molding methylpentene copolymers and polycarbonates with the mold. The size of the cover was 81 mm × 123 mm × 0.4 mm, and the size of the container was 81 mm × 123 mm × 1.6 mm. 384 wells with a trapezoidal cross section were formed on the surface of the container, their size being such that the diameter of the mouth portion was 1.3 mm, the diameter of the bottom portion was 1.1 mm and the depth was 0.8 mm (volume 0.9 µL/well), or the diameter of the mouth portion was 1.1 mm, the diameter of the bottom portion was 0.9 mm and the depth was 0.03 mm (volume 0.02 µL/well). The height of the raised portions was 0.4 mm, with an inner diameter of 1.4 mm and outer diameter of 2.4 mm, and the runoff channels had an inner diameter of 3.0 mm an outer diameter of 4.0 mm and a depth of 0.6 mm The height of the liquid expelling portions formed on the cover was 0.2 mm, with an outer diameter of 0.9 mm.

First, 1 µL or 0.2 µL of 384 different types of genomic DNA (40 ng/µL) was injected by means of a spotting apparatus into the respective wells of the container placed on an experimental stand. After leaving the container in the atmosphere and allowing the solvent to evaporate, roughly 1.6 µL or 0.2 µL of a reagent for the Invader process having a fluorescence intensity peak at a wavelength of 570 nm was injected into each well by means of the spotting apparatus. After injecting the reagent, the cover was pressed against the wells so as to expel the extra reagent bulging out from the wells, and the liquid in the wells was sealed. Then, the raised portions at the mouth portions of the wells were welded to the cover by means of an ultrasonic apparatus. In the liquid-proof state, the DNA was denatured for 10 minutes at 95 °C, after which it was allowed to react for 4 hours in an isothermic bath of 63 °C. After the reaction, the fluorescent light intensity was measured by a plate reader. The tightness of the seals on the wells was good, with no air bubbles being apparent, neither immediately after welding nor after the reaction of 4 hours. An ultrasonic welding apparatus wherein the frequency of the ultrasonic waves was held at 20 kHz, the amplitude of the horns was 36 microns, and the maximum oscillation output was 5.0 kW was used. The welding time, vibration pressure and well volume were changed as shown in Table 1. The results of the measurements are shown in Table 1. As is clear from Table 1, when the well volume was 0.02 µL, the quantity of liquid was too small, thus reducing the reactivity so that no fluorescent light was detected.

Comparative Examples 4-10

Experiment

10 ng, 20 ng, 40 ng and 100 ng of genomic DNA were injected into a 384-well microtiter plate, and either 20 µL or 40 µL of a reagent of the Invader process (having a fluorescent light intensity peak at a wavelength of 570 nm) was injected into each well. In order to make the wells liquid-proof, they were capped, and after denaturing the DNA for 5 minutes at 95 °C, they were allowed to react for four hours in a thermal cycler set to 63 °C, and after the reaction, the fluorescent light intensity was measured with a plate reader. The results are shown in Table 1.

Comparative Example 11

Experiment

10 ng of genomic DNA were injected in at 384-well microtiter plate, after which 20 µL of a TaqMan process reagent (having a fluorescent light intensity peak at a wavelength of 570 nm) was injected in each well. In order to make the wells liquid-proof, they were capped, and after denaturing the DNA for 10 minutes at 95 °C, they were put into an incubation cycle 1 minute at 95 °C and 3 minutes at 60 °C repeated 40 times. After the reaction, the fluorescent light intensity was measured in a plate reader. The results are shown in Table 1.

(Examples 20-21 and Comparative Examples 12-13 relating to Ultrasonic Welding Experiment)

Experiment

A cover and container were produced by making a mold, then injection molding methylpentene copolymers and polycarbonates with the mold. The size of the cover was 81 mm $\times$ 123 mm $\times$ 0.4 mm, and the size of the container was 81 mm $\times$ 123 mm $\times$ 1.6 mm. 384 wells with a trapezoidal cross section were formed on the surface of the container, their size being such that the diameter of the mouth portion was 1.3 mm, the diameter of the bottom portion was 1.1 mm and the depth was 0.8 mm (volume 0.9 $\mu$L/well). The height of the raised portions was 0.4 mm, with an inner diameter of 1.4 mm and outer diameter of 2.4 mm, and the runoff channels had an inner diameter of 3.0 mm, an outer diameter of 4.0 mm and a depth of 0.6 mm. The height of the liquid expelling portions formed on the cover was 0.2 mm, with an outer diameter of 0.9 mm.

First, 1 $\mu$L of water was injected by means of a spotting apparatus into the respective wells of the container placed on an experimental stand. After injecting the water, the cover was pressed against the wells so as to expel the extra reagent bulging out from the wells, and the liquid in the wells was sealed. Then, the raised portions at the mouth portions of the wells were welded to the cover by means of an ultrasonic apparatus. An ultrasonic welding apparatus with a ultrasonic frequency of 20 kHz and a maximum oscillation output of 5.0 kW was used. The maximum oscillation output during welding was 4.1-5.0 kW. Thereafter, the result was heated for 5 minutes 95 °C, then left for 4 hours in an isothermic bath of 63 °C. The tightness of the seals on the wells was good, with no air bubbles being apparent, neither immediately after welding nor after the reaction of 4 hours. The welding time, oscillation pressure and horn amplitude were varied as shown in Table 2. As is clear from Table 2, it is most preferable to set the horn amplitude to 30-40 $\mu$m in order to make the wells liquid-tight

As is clear from Tables 1 and 2, by using a microwell array composed of methylpentene copolymers or polycarbonates, it is possible to obtain the same level of fluorescent light intensity as in conventional microtiter plates, with approximately one-tenth the amount of DNA and reagent. Additionally, by optimizing the welding time and vibration pressure, it is possible to obtain stable measurements. Additionally, by placing a reflective body behind the microwell array, the intensity of detectable fluorescent light was able to be roughly doubled.

As described above, according to the present invention, liquid in an amount roughly equal to or exceeding the volume of a well after welding is spotted in wells, and the liquid is pushed out of the wells by means of a cover or intermediary body, thus enabling the liquid to be sealed with almost no air residing in the wells. Thus, by trapping a fluorescent light reagent in a minuscule space, all of the fluorescent light reagent can be effectively excited, and the emitted fluorescent light can be efficiently detected by making use of a light reflecting body. While the examples explained here have been of the 384-well type, the above-described invention can of course be applied to various types of microwell arrays, such as those with 1536 wells or 9600 wells.

Table 1

| | | Gen. DNA | Well Vol. | Invader Reagent | Int. after 4 h | Weld Time | Vib. Press. | Corresponding Drawing |
|---|---|---|---|---|---|---|---|---|
| | | no./well | µL | µL/well | (relative) | sec | N/cm | |
| Ex. 1 | Microwell Array | 10 | 0.9 | 1.6 | 43 | 0.25 | 50 | Fig. 1 |
| Ex. 2 | | 20 | 0.9 | 1.6 | 91 | 0.25 | 50 | Fig. 1 |
| Ex. 3 | | 40 | 0.9 | 1.6 | 190 | 0.25 | 50 | Fig. 1 |
| Ex. 4 | | 40 | 0.9 | 1.6 | 205 | 0.05 | 50 | Fig. 1, weld time |
| Ex. 5 | | 40 | 0.9 | 1.6 | 165 | 0.8 | 50 | Fig. 1, weld time |
| Ex. 6 | | 40 | 0.9 | 1.6 | 211 | 0.25 | 0.3 | Fig. 1, vib. press. |
| Ex. 7 | | 40 | 0.9 | 1.6 | 195 | 0.25 | 100 | Fig. 1, vib. press. |
| Ex. 8 | | 40 | 0.9 | 1.6 | 375 | 0.25 | 50 | Fig. 1, reflector |
| Ex. 9 | | 40 | 0.1 | 0.2 | 41 | 0.25 | 50 | Fig. 1 |
| Ex. 10 | | 10 | 1.1 | 1.6 | 48 | 0.25 | 50 | Fig. 14 + Fig. 17 |
| Ex. 11 | | 20 | 1.1 | 1.6 | 100 | 0.25 | 50 | Fig. 14 + Fig. 17 |
| Ex. 12 | | 40 | 1.1 | 1.6 | 212 | 0.25 | 50 | Fig. 14 + Fig. 17 |
| Ex. 13 | | 10 | 1.1 | 2.0 | 46 | 0.25 | 50 | Fig. 14 + Fig. 17 |
| Ex. 14 | | 20 | 1.1 | 2.0 | 102 | 0.25 | 50 | Fig. 14 + Fig. 17 |
| Ex. 15 | | 40 | 1.1 | 2.0 | 220 | 0.25 | 50 | Fig. 14 + Fig. 17 |
| Ex. 16 | | 10 | 1.4 | 2.0 | 43 | 0.25 | 50 | Fig. 14 + Fig. 17 |
| Ex. 17 | | 20 | 1.4 | 2.0 | 92 | 0.25 | 50 | Fig. 14 + Fig. 17 |
| Ex. 18 | | 40 | 1.4 | 2.0 | 189 | 0.25 | 50 | Fig. 14 + Fig. 17 |
| Ex. 19 | | 10 | 0.9 | 1.6 | 46 | 0.25 | 50 | Fig. 1, TaqMan |
| Co. Ex. 1 | | 40 | 0.9 | 1.6 | 2 | 0.9 | 50 | Fig. 1 |
| Co. Ex. 2 | | 40 | 0.9 | 1.6 | 0 | 0.25 | 0.1 | Fig. 1 |
| Co. Ex. 3 | | 40 | 0.02 | 0.2 | 0 | 0.25 | 50 | Fig. 1 |
| Co. Ex. 4 | Microtiter Plate | 10 | 40 | 20 | 5 | - | - | |
| Co. Ex. 5 | | 20 | 40 | 20 | 11 | - | - | |
| Co. Ex. 6 | | 40 | 40 | 20 | 23 | - | - | |
| Co. Ex. 7 | | 100 | 40 | 20 | 53 | - | - | |
| Co. Ex. 8 | | 20 | 40 | 40 | 4 | - | - | |
| Co. Ex. 9 | | 40 | 40 | 40 | 13 | - | - | |
| Co. Ex. 10 | | 100 | 40 | 40 | 27 | - | - | |
| Co. Ex. 11 | | 10 | 40 | 20 | 6 | - | - | TaqMan |

Table 2

| | Well Cap. | Injected Water Vol. | Horn Amp. | Weld Time | Osc. Press. | Total Press. | Well Water Vol. after Weld | Corr. Draw. |
|---|---|---|---|---|---|---|---|---|
| | µL | µL | µm | sec | N/cm | N | µL | |
| Ex. 20 | 0.9 | 1.0 | 30 | 0.25 | 50 | 10000 | 0.9 | Fig. 1 |
| Ex. 21 | 0.9 | 1.0 | 40 | 0.25 | 50 | 10000 | 0.9 | Fig. 1 |
| Co. Ex. 12 | 0.9 | 1.0 | 28 | 0.25 | 50 | 10000 | bad weld | Fig. 1 |
| Co. Ex. 13 | 0.9 | 1.0 | 42 | 0.25 | 50 | 10000 | 0.0 | Fig. 1 |

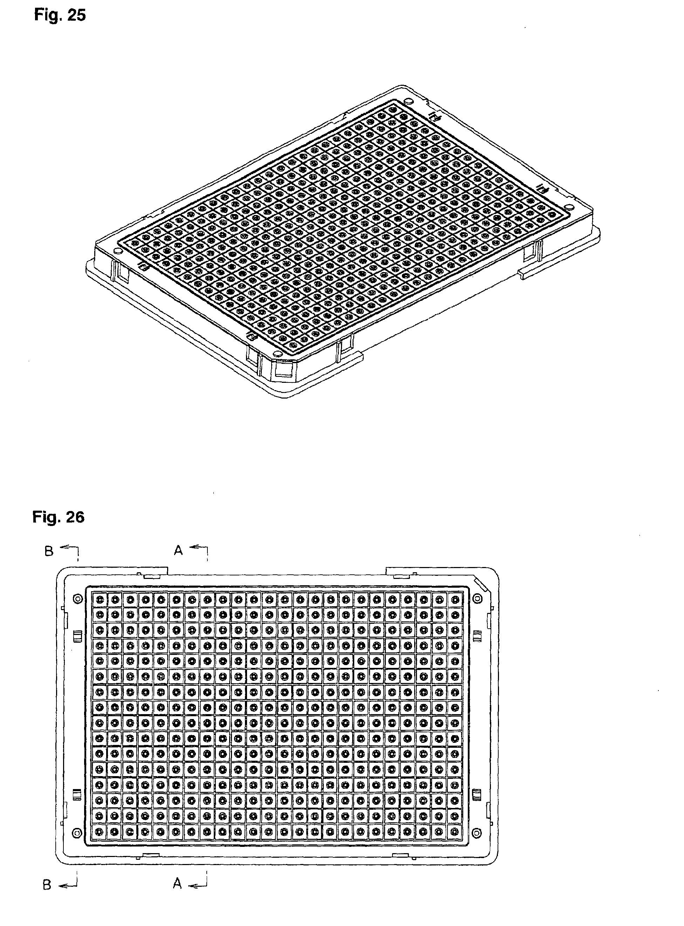

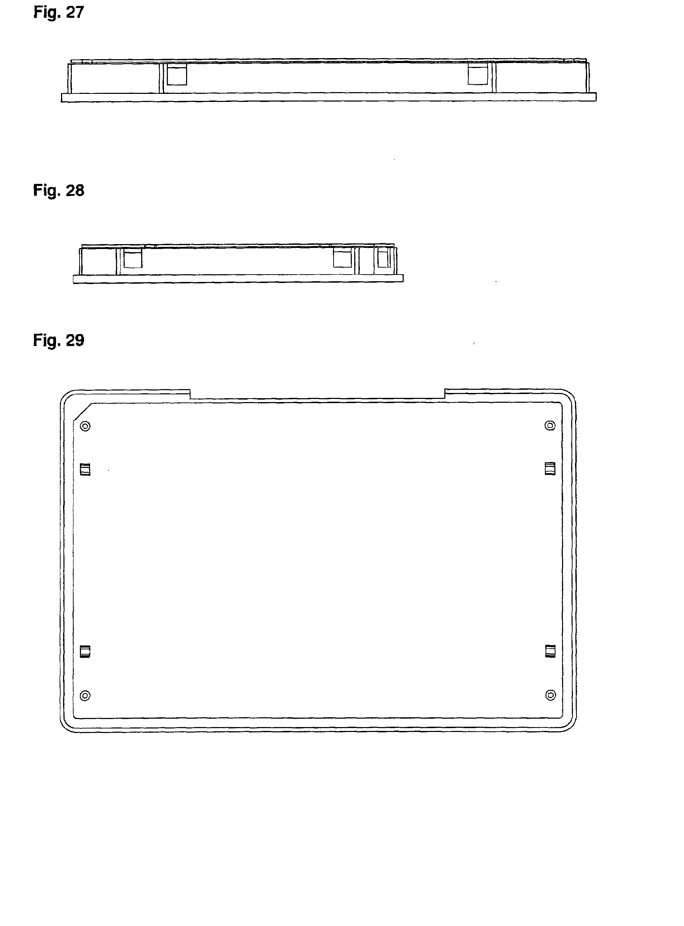

Figs. 25 through 34 are drawings showing an embodiment of a microwell array according to the present invention. As shown in Fig. 15, this microwell array is roughly planar, with microwells arranged at regular intervals in the XY directions. Whereas the drawing assumes that the cover is a transparent cover, it does not necessarily need to be transparent. However, it is favorable for the purposes of light detection for at least one of the cover or the main body to be composed of a material that transmits light. Fig. 25 is a diagram showing the embodiment of the microwell array shown in Fig. 25 as seen from above. As is clear from the drawing, 384 microwells are formed in this embodiment, with channels formed between the microwells on the main body so as to contain the runoff from the microwells to prevent cross-contamination. Figs. 27 and 28 are side views of the microwell array shown in Fig. 25. While the microwell has a certain height in consideration of the need to maintain the strength of the microwell array and the convenience when stacking them for storage, but this height is determined only by the vertical walls on the periphery, and the portions underneath aside form the peripheral portions are hollow. Fig. 29 is a bottom view of the microwell array as seen from below.

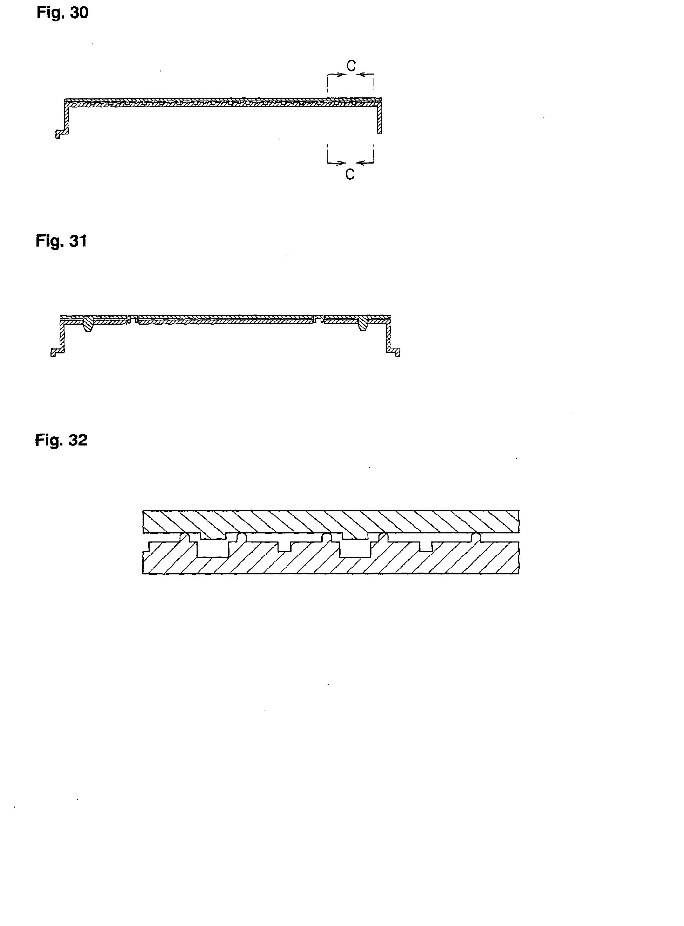

Fig. 30 shows a section vie of the portion indicated by AA of the microwell array as indicated in Fig. 25. This shows the main body and cover in a combined state, with the portion beneath the central portion of the main body being hollow. Fig. 31 is a section view of the portion indicated by BB in Fig. 25, from which it can be seen that positioning is accomplished by projections provided in the cover and through holes bored through the main body. Fig. 32 is an enlarged section view of the portion indicated by CC in Fig. 30. The positional relationship between the raised portions formed in the periphery of the microwell array and the projecting portions formed in the cover are clearly shown. Fig. 33 is a drawing showing the bottom surface of the cover of the microwell array shown in Fig. 25. In the case of the present embodiment, projections are formed at positions corresponding to the microwells. Fig. 34 shows the cover as seen from the side.

Fig. 35 shows a perspective view of another embodiment of the present invention which is the same as the embodiment given above with regard to having 384 microwells, but does not have channels between the microwells. As is clear from Fig. 36 and 37, it differs in not having channels formed on the main body, but is the same as the previous embodiment with respect to all other points.

Fig. 38 is a perspective view of yet another embodiment of the microwell array having 96 microwells. The lateral dimensions and outward shape are the same as the above embodiments, but the size and number of microwells formed in the main body are different. By standardizing the shape and outer form, the work efficiency can be improved through sharing of various types of apparatus for handling microwell arrays.

Fig. 39 shows an embodiment of a microwell array which, as opposed to the previous embodiment, has an extremely large number of microwells. In this case as well, the work efficiency can be improved by standardizing the shape and outer form, but it should be self-evident that there is no need to restrict the shape to that shown in the drawing.

While the above-described drawings show some possible embodiments in relative detail for giving specific images of the microwell arrays based on the present invention, those skilled in the art will recognize that there is absolutely no need to use the forms given above in order to achieve the technical concepts of the present invention, and countless variations are possible aside from the forms described. Accordingly, the scope of the present invention is such as to include all variations and modifications which retain the claimed features, as well as their equivalents.

INDUSTRIAL APPLICABILITY

According to the present invention, liquid of a quantity roughly equal to or more than the capacity of the wells after welding can be spotted in the wells, and if the liquid exceeds the capacity of the wells, the extra liquid can be expelled from the wells by a cover and intermediary portions, enabling the liquid to be sealed with almost no air left in the wells, thereby enabling the fluorescent light that acts as a signal to be efficiently detected.

**Claims**

1.    A microwell array comprising a container with a plurality of isolated wells positioned in an array, and a cover capable of covering the container; wherein raised portions which are higher than the surrounding portions are provided at the peripheral portions of each well.

2.    A microwell array comprising a container with a plurality of isolated wells positioned in an array, and a cover capable of covering the container; wherein raised portions for welding which are higher than the surrounding portions are provided at the peripheral portions of each well.

3.    A microwell array comprising a container with a plurality of isolated wells positioned in an array, and a cover capable of covering the container; wherein raised portions which are higher than the surrounding portions are provided on the cover at positions corresponding to the peripheral portions of each well when the container is covered by the cover.

4. A microwell array comprising a container with a plurality of isolated wells positioned in an array, and a cover capable of covering the container; wherein raised portions for welding which are higher than the surrounding portions are provided on the cover at positions corresponding to the peripheral portions of each well when the container is covered by the cover.

5. A microwell array in accordance with any one of claims 1-4, wherein said raised portions are annular, and their vertex portions are convex.

6. A microwell array comprising a container with a plurality of isolated wells positioned in an array, and a cover capable of covering the container; wherein channels for catching liquid overflowing from the wells when the wells are covered by the cover are formed in at least one of an area surrounding each well or the positions on the cover corresponding to said area surrounding each well.

7. A microwell array comprising a container with a plurality of isolated wells positioned in an array, and a cover covering the container; wherein said cover and said wells containing liquid are welded together at each well.

8. A microwell array comprising a container with a plurality of isolated wells positioned in an array, and a cover covering the container; wherein said cover and said wells containing liquid are ultrasonically welded together at each well.

9. A microwell array comprising a container with a plurality of isolated wells positioned in an array, and a cover covering the container and sealing each well in a liquid-tight manner, wherein the substantial thicknesses of the container and cover are both of a thickness such as to be able to transmit the heat of liquid contacting said container and said cover to the insides of the wells.

10. A microwell array comprising a container with a plurality of isolated wells positioned in an array, and a cover covering the container and sealing each well in a liquid-tight manner, wherein the substantial thicknesses of the container and cover are both within the range of 0.15-3.0 mm.

11. A microwell array comprising a container with a plurality of isolated wells positioned in an array, and a cover covering the container; wherein the substantial thickness of the microwell array when said well and said cover are welded in a liquid-tight manner at each well is within the range of 0.3-4.0 mm.

12. A microwell array comprising a container with a plurality of isolated wells positioned in an array, and a cover covering the container; wherein the thickness of the portions directly defining the wells when said well and said cover are welded in a liquid-tight manner at each well is within the range of 0.05-0.4 mm.

13. A microwell array comprising a container with a plurality of isolated wells positioned in an array, and a cover covering the container and sealing each well in a liquid-tight manner, wherein welding ribs having sufficient volume for welding said well s and said cover are provided in the vicinity of said wells.

14. A microwell array comprising a container with a plurality of isolated wells positioned in an array, and a cover covering the container and sealing each well in a liquid-tight manner, wherein welding ribs having a triangular cross-section are provided in the vicinity of said wells.

15. A microwell array comprising a container with a plurality of isolated wells positioned in an array, and a cover covering the container and sealing each well in a liquid-tight manner, wherein the thicknesses of the bottoms of welding ribs in the vicinity of said wells are within the range of 0.2-1.0 mm.

16. A microwell array comprising a container with a plurality of isolated wells positioned in an array, and a cover covering the container and sealing each well in a liquid-tight manner, wherein the thicknesses of the bottoms of welding ribs in the vicinity of said wells are within the range of 0.2-1.0 mm, their heights are within the range of 0.2-0.8 mm, and the diameters of ribs surrounding said wells are within the range of 0.5-4.0 mm.

17. A microwell array comprising a container with a plurality of isolated wells positioned in an array, and a cover covering the container and sealing each well in a liquid-tight manner, wherein the widths of welding portions surrounding the wells for joining said wells and said cover at each well in a liquid-tight manner are within the range of 0.3-2.5 mm.

18. A microwell array comprising a container with a plurality of isolated wells positioned in an array, and a cover capable of covering the container; wherein said wells have a capacity such that the liquid temperature inside said wells becomes uniform within a few minutes upon immersion of said microwell array in an isothermic bath.

19. A microwell array comprising a container with a plurality of isolated wells positioned in an array, and a cover capable of covering the container; wherein the capacity of said wells is within the range of 0.1-1.4 µl.

20. A microwell array comprising a container with a plurality of isolated wells positioned in an array, and a cover capable of covering the container; wherein the capacity of said wells when said wells and said cover are joined in a liquid-tight manner at each well is within the range of 0.1-1.4 µl.

21. A microwell array comprising a container with a plurality of isolated wells positioned in an array, and a cover covering the container; wherein said well and said cover seal the wells in a liquid-tight manner, and the seal of the wells is maintained even if the liquid boils inside the wells.

22. A microwell array in accordance with claim 16, wherein the seal of said wells is obtained by ultrasonically welding said wells and said cover.

23. A microwell array comprising a container with a plurality of isolated wells positioned in an array, and a cover covering the container; wherein said well and said cover seal the wells in a liquid-tight manner, and the seal of the wells is maintained without applying any external mechanical forces even if the liquid boils inside the wells.

24. A microwell array comprising a container with a plurality of isolated wells positioned in an array, and a cover covering the container; wherein said well and said cover seal the wells in a liquid-tight manner, and the seal of the wells is maintained even if a liquid heated to 90-100 °C boils inside the wells.

25. A microwell array comprising a container with a plurality of isolated wells positioned in an array, and a cover covering the container; wherein said well and said cover seal the wells in a liquid-tight manner, and the seal of the wells is maintained even if said microwell array is immersed in a boiling liquid.

26. A microwell array in accordance with any one of claims 1-25, wherein an intermediary body which is roughly planar and composed of a material having flexibility is placed between the container and the cover.

27. A microwell array comprising a container with a plurality of isolated wells positioned in an array, and a cover capable of covering the container; wherein a convex portion which is pushed into each well when sealing the well by means of the cover is formed at a position of the cover corresponding to each well when the container is covered by the cover.

28. A method of sealing liquid comprising steps of injecting fluid into wells of a container having a plurality of isolated wells positioned in an array, covering the container with a cover, applying pressure to make the container and cover come into tight contact, next irradiating with ultrasonic waves while applying said pressure so as to ultrasonically weld said wells and said cover at each well such that the liquid in the wells of said container does not spill out.

29. A liquid sealing method comprising steps of using a container having a plurality of isolated wells positioned in an array and a cover capable of covering the container, injecting liquid into the wells, pressing the cover onto the container, then welding together said cover and said container to seal said liquid into each well.

30. A method in accordance with either claim 29 or 30, wherein said liquid is a liquid containing DNA or proteins.

31. A liquid sealing method in accordance with any one of claims 29-30 wherein said welding is performed by ultrasonic welding.

32. A method in accordance with claim 28 or 31, wherein the ultrasonic waves used for said ultrasonic welding substantially do not damage the DNA or proteins sealed inside said wells.

33. A liquid sealing method in accordance with claim 28 or 31, wherein the wells of said container and said cover are welded by ultrasonic vibrations lasting 0.05 to 0.8 seconds for a liquid-tight seal.

34. A liquid sealing method in accordance with any one of claims 28, 31 or 32 comprising a step of starting the vibration of the ultrasonic horn while applying a force of 0.3 to 100 N per 1 cm of length of a raised portion to be welded when performing said welding.

35. A microwell array in accordance with any one of claims 1-27, wherein said container and said cover are composed of materials capable of sealing off each well by means of ultrasonic welding.

36. A microwell array in accordance with any one of claims 1-27 and 35, wherein the rear surface of each well is planar.

37. A microwell array in accordance with any one of claims 1-27, 35 and 36, wherein a reflective surface for reflecting light is provided above or below said wells.

38. A liquid injecting method using a microwell array in accordance with any one of claims 1-27 and 35-37, comprising steps of first distributively injecting a liquid by means of a contact-type distributive injector, then distributively injecting a liquid by means of a non-contact-type distributive injector.

39. A liquid injecting method in accordance with claim 38, comprising steps of first distributively injecting a different liquid in each well by means of a contact-type distributive injector, then distributively injecting a liquid by means of a non-contact-type distributive injector.

40. A liquid injecting method in accordance with either claim 38 or 39, comprising steps of first distributively injecting liquid by means of a contact-type distributive injector, drying said liquid, then distributively injecting a liquid by means of a non-contact-type distributive injector.

41. A microwell array in accordance with any one of claims 1-27 and 35-37, wherein the wells have a circular horizontal cross section.

42. A microwell array in accordance with any one of claims 1-27, 35-36 and 41, wherein a skirt portion is formed along the outer peripheral portion of said microwell array.

43. A microwell array in accordance with claim 42, wherein through holes are formed in the comers of the skirt portion formed on said microwell array.

44. A microwell array in accordance with any one oc claims 1-27, 35-37 and 41-43, wherein an insertion portion and receiving portion are formed on said container and said cover, and said container and said cover can be engaged by fitting the insertion portion into the receiving portion.

45. A microwell array in accordance with any one of claims 1-27, 35-37 and 41-44, wherein said container and said cover are formed of a plastic material.

46. A microwell array in accordance with claim 45, wherein said container and said cover are composed of a methyl pentene copolymer or polycarbonate.

47. A microwell array in accordance with any one of claims 1-27, 35-37 and 41-46, wherein at least one of said container and said cover is formed of an optically transparent material.

48. A microwell array in accordance with any one of claims 1-27, 35-37 and 41-47, wherein a liquid reagent or sample is distributively injected and held in the wells of said container or on the surface on the well side of said cover.

49. A microwell array in accordance with any one of claims 1-27, 35-37 and 41-49, wherein a liquid reagent or sample is distributively injected by a non-contact-type distributive injector and held in the wells of said container or on the surface on the well side of said cover.

50. A method of using a microwell array in accordance with any one of claims 1-27, 35-37 and 41-49, wherein at least one of a reagent or sample is held in the wells of said container, the other is held on the surface of the cover, and said wells and said cover are joined by ultrasonic welding to induce a reaction between the reagent and sample in each well.

**51.** A method for producing a microwell array in accordance with any one of claims 1-27, 35-37 and 41-50, wherein resin is poured in from a side gate for injection molding of said container and said cover.

**52.** An ultrasonic welding apparatus capable of applying a force of 7000-23000 N during oscillation, and having a maximum oscillation output of 4.1-5.0 kW.

**53.** An ultrasonic welding apparatus wherein the horn amplitude is 30-40 microns, capable of applying a force of 7000-23000 N during oscillation, and having a maximum oscillation output of 4.1-5.0 kW.

**54.** An ultrasonic welding apparatus wherein the horn amplitude is 30-40 microns, capable of applying a force of 7000-23000 N during oscillation, having a maximum oscillation output of 4.1-5.0 kW, and being capable of emitting ultrasonic waves within a welding time of 0.05-0.8 seconds.

**55.** A sealing method and ultrasonic welding apparatus capable of sealing, within 0.05-0.8 seconds, each well in a microwell array as recited in any one of claims 1-23, 31-33 and 37-46, by means of an ultrasonic welding apparatus wherein the hom amplitude is 30-40 microns, capable of applying a force of 7000-23000 N during oscillation, and having a maximum oscillation output of 4.1-5.0 kW.

**56.** A sealing and distributive injecting method, comprising steps of distributively injecting a reagent or sample into well portions or cover surfaces of a microwell array comprising a container having a plurality of isolated wells arranged in an array, and a cover capable of covering the container, then welding together said cover and said wells so that each well is liquid-tight.

**57.** An analysis method comprising steps of distributively injecting a reagent or sample into well portions or cover surfaces of a microwell array comprising a container having a plurality of isolated wells arranged in an array, and a cover capable of covering the container, then welding together said cover and said wells so that each well is liquid-tight, and performing fluorescent light intensity analysis for each well after enabling the reagent and sample to react, or while enabling the reagent and sample to react.

**58.** A genetic analysis method comprising steps of distributively injecting a reagent or sample into well portions or cover surfaces of a microwell array comprising a container having a plurality of isolated wells arranged in an array, and a cover capable of covering the container, then welding together said cover and said wells so that each well is liquid-tight, and performing fluorescent light intensity analysis for each well after enabling the reagent and sample to react, or while enabling the reagent and sample to react, thereby to analyze the genes of each well.

**59.** A genetic polymorphism analysis method comprising steps of distributively injecting a reagent or sample into well portions or cover surfaces of a microwell array comprising a container having a plurality of isolated wells arranged in an array, and a cover capable of covering the container, then welding together said cover and said wells so that each well is liquid-tight, and performing fluorescent light intensity analysis for each well after enabling the reagent and sample to react, or while enabling the reagent and sample to react, thereby to analyze the genetic polymorphism of each well.

**60.** An analysis method comprising steps of appending a bar code corresponding to each reagent and sample distributively injected into a microwell array comprising a container having a plurality of isolated wells arranged in an array, and a cover capable of covering the container, enabling the progress to be managed by the bar code for each step or each microwell array, then welding together said cover and said wells so that each well is liquid-tight, and performing fluorescent light intensity analysis for each well after enabling the reagent and sample to react, or while enabling the reagent and sample to react, thereby to analyze at least one of the degree of the reaction, genes and genetic polymorphism for each well.

**61.** A genetic polymorphism analysis method comprising steps of distributively injecting different DNA into each well of a microwell array comprising a container having a plurality of isolated wells arranged in an array, and a cover capable of covering the container, next distributively injecting reagent into the plurality of said wells, then welding together said wells and said cover, enabling the reagent to react with the DNA, and analyzing the fluorescent light intensity of each well to perform polymorphic typing.

**62.** A genetic polymorphism analysis method comprising steps of distributively injecting a reagent into each well of a microwell array comprising a container having a plurality of isolated wells arranged in an array, and a cover capable of covering the container, next distributively injecting different DNA into the plurality of said wells, then welding together said wells and said cover, enabling the reagent to react with the DNA, and analyzing the fluorescent light intensity of each well to perform polymorphic typing.

63. A genetic polymorphism analysis method comprising steps of distributively injecting a reagent a cover surface of a microwell array comprising a container having a plurality of isolated wells arranged in an array, and a cover capable of covering the container, next distributively injecting different DNA into the plurality of said wells, then welding together said wells and said cover, enabling the reagent to react with the DNA, and analyzing the fluorescent light intensity of each well to perform polymorphic typing.

64. A genetic diagnosis method comprising steps of distributively injecting different reagents onto a cover surface of a microwell array comprising a container having a plurality of isolated wells arranged in an array, and a cover capable of covering the container, next distributively injecting different DNA into the plurality of said wells, then welding together said wells and said cover, enabling the reagent to react with the DNA, and analyzing the fluorescent light intensity of each well to perform genetic polymorphism analysis.

65. A genetic diagnosis method comprising steps of distributively injecting different reagents into the wells of a microwell array comprising a container having a plurality of isolated wells arranged in an array, and a cover capable of covering the container, next distributively injecting different DNA into the plurality of said wells, then welding together said wells and said cover, enabling the reagent to react with the DNA, and analyzing the fluorescent light intensity of each well to perform genetic polymorphism analysis.

**Amended claims under Art. 19.1 PCT**

1. A microwell array comprising a container with a plurality of isolated wells positioned in an array, and a cover capable of covering the container; wherein the cover is welded to the container with liquid injected into the wells.

2. A microwell array in accordance with claim 1, wherein raised portions which are higher than the surrounding portions prior to welding are provided at the peripheral portions of each well, or at positions on the cover corresponding to the peripheral portions of each well when the container is covered by the cover.

3. A microwell array in accordance with claim 2, wherein said raised portions are raised portions for welding.

4. A microwell array in accordance with either of claims 2 or 3, wherein said raised portions are annular, and their vertex portions are convex.

5. A microwell array in accordance with any one of claims 1-4, wherein channels for catching liquid overflowing from the wells when the wells are covered by the cover are formed in at least one of an area surrounding each well or the positions on the cover corresponding to said area surrounding each well.

6. A microwell array in accordance with any one of claims 1-5, wherein said cover and said wells containing liquid are welded together at each well.

7. A microwell array in accordance with any one of claims 1-6, wherein said welding is performed by ultrasonic welding.

8. A microwell array in accordance with any one of claims 1-7, wherein the cover seals each well in a liquid-tight manner, and the substantial thicknesses of the container and cover are both of a thickness such as to be able to transmit the heat of liquid contacting said container and said cover to the insides of the wells.

9. A microwell array in accordance with claim 8, wherein the substantial thicknesses of the container and cover are both within the range of 0.15-3.0 mm.

10. A microwell array in accordance with any one of claims 1-9, wherein the substantial thickness of the microwell array when said well and said cover are welded in a liquid-tight manner at each well is within the range of 0.3-4.0 mm.

11. A microwell array in accordance with any one of claims 1-10, wherein the thickness of the portions directly defining the wells when said well and said cover are welded in a liquid-tight manner at each well is within the range of 0.05-0.4 mm.

12. A microwell array in accordance with any one of claims 1-11, wherein the cover seals each well in a liquid-tight manner, and welding ribs having sufficient volume for welding said well s and said cover are provided in the vicinity of said wells.

13. A microwell array in accordance with claim 12, wherein the welding ribs have a triangular cross-section.

14. A microwell array in accordance with claim 12 or 13, wherein the thicknesses of the bottoms of the welding ribs are within the range of 0.2-1.0 mm.

15. A microwell array in accordance with any one of claims 12-14, wherein the thicknesses of the bottoms of the welding ribs are within the range of 0.2-1.0 mm, their heights are within the range of 0.2-0.8 mm, and the diameters of ribs surrounding said wells are within the range of 0.5-4.0 mm.

16. A microwell array in accordance with any one of claims 1-15 wherein the cover seals each well in a liquid-tight manner, and the widths of welding portions surrounding the wells for joining said wells and said cover at each well in a liquid-tight manner are within the range of 0.3-2.5 mm.

17. A microwell array in accordance with any one of claims 1-16, wherein said wells have a capacity such that the liquid temperature inside said wells becomes uniform within a few minutes upon immersion of said microwell array in an isothermic bath.

18. A microwell array in accordance with claim 17, wherein the capacity of said wells is within the range of 0.1-1.4 $\mu$l.

19. A microwell array in accordance with any one of claims 1-18, the capacity of said wells when said wells and said cover are joined in a liquid-tight manner at each well is within the range of 0.1-1.4 $\mu$l.

20. A microwell array in accordance with any one of claims 1-19, wherein said well and said cover seal each well in a liquid-tight manner, and the seal of the wells is maintained even if the liquid boils inside the wells.

21. A microwell array in accordance with claim 20, wherein the seal of the wells is maintained without applying any external mechanical forces even if the liquid boils inside the wells.

22. A microwell array in accordance with either claim 20 or 21, wherein the seal of said wells is obtained by ultrasonically welding said wells and said cover.

23. A microwell array in accordance with any one of claims 20-22, wherein the seal of the wells is maintained even if a liquid heated to 90-100 °C boils inside the wells.

24. A microwell array in accordance with any one of claims 20-23, wherein the seal of the respective wells is maintained even if said microwell array is immersed in a boiling liquid.

25. A microwell array in accordance with any one of claims 1-24, wherein an intermediary body which is roughly planar and composed of a material having flexibility is placed between the container and the cover.

26. A microwell array in accordance with any one of claims 1-25, a convex portion which is pushed into each well when sealing the well by means of the cover is formed at a position of the cover corresponding to each well when the container is covered by the cover.

27. A microwell array in accordance with any one of claims 1-26, wherein said container and said cover are composed of materials capable of sealing off each well by means of ultrasonic welding.

28. A microwell array in accordance with any one of claims 1-27, wherein the rear surface of each well is planar.

29. A microwell array in accordance with any one of claims 1-28, wherein the wells have a circular horizontal cross section.

**30.** A microwell array in accordance with any one of claims 1-29, wherein a skirt portion is formed along the outer peripheral portion of said microwell array.

**31.** A microwell array in accordance with claim 30, wherein through holes are formed in the corners of the skirt portion formed on said microwell array.

**32.** A microwell array in accordance with any one of claims 1-31, wherein an insertion portion and receiving portion are formed on said container and said cover, and said container and said cover can be engaged by fitting the insertion portion into the receiving portion.

**33.** A microwell array in accordance with any one of claims 1-32, wherein said container and said cover are formed of a plastic material.

**34.** A microwell array in accordance with claim 33, wherein said container and said cover are composed of a methyl pentene copolymer or polycarbonate.

**35.** A microwell array in accordance with any one of claims 1-34, wherein at least one of said container and said cover is formed of an optically transparent material.

**36.** A microwell array in accordance with any one of claims 1-35, wherein a reflective surface for reflecting light is provided above or below said wells.

**37.** A microwell array in accordance with any one of claims 1-36, wherein a liquid reagent or sample is distributively injected and held in the wells of said container or on the surface on the well side of said cover.

**38.** A microwell array in accordance with any one of claims 1-37, wherein a liquid reagent or sample is distributively injected by a non-contact-type distributive injector and held in the wells of said container or on the surface on the well side of said cover.

**39.** A microwell array in accordance with any one of claims 1-38, wherein said liquid is a liquid containing DNA or proteins.

**40.** A method for manufacturing a microwell array comprising forming a container having a plurality of isolated wells positioned in an array and a cover capable of covering the container, injecting liquid into the wells, pressing the cover onto the container then welding together said cover and said container to seal said liquid into each well.

**41.** A manufacturing method in accordance with claim 40, wherein said welding is performed by ultrasonic welding.

**42.** A manufacturing method in accordance with claim 40 or 41, comprising injecting fluid into the wells, covering the container with the cover, applying pressure to make the container and cover come into tight contact, next irradiating with ultrasonic waves while applying said pressure so as to ultrasonically weld said wells and said cover at each well such that the liquid in the wells of said container does not spill out.

**43.** A manufacturing method in accordance with any one of claims 40-42, wherein said liquid is a liquid containing DNA or proteins.

**44.** A manufacturing method in accordance with claim 43, wherein the ultrasonic waves used for said ultrasonic welding substantially do not damage the DNA or proteins sealed inside said wells.

**45.** A manufacturing method in accordance with any one of claims 40-44, wherein the wells of said container and said cover are welded by ultrasonic vibrations lasting 0.05 to 0.8 seconds for a liquid-tight seal.

**46.** A manufacturing method in accordance with any one of claims 40-45 comprising starting the vibration of the ultrasonic horn while applying a force of 0.3 to 100 N per 1 cm of length of a raised portion to be welded when performing said welding.

**47.** A manufacturing method in accordance with any one of claims 40-46, comprising distributively injecting a liquid by means of a contact-type distributive injector, then distributively injecting a liquid by means of a non-contact-type distributive injector.

48. A manufacturing method in accordance with claim 47, comprising distributively injecting a different liquid in each well by means of a contact-type distributive injector, then distributively injecting a liquid by means of a non-contact-type distributive injector.

49. A manufacturing method in accordance with either claim 47 or 48, comprising distributively injecting liquid by means of a contact-type distributive injector, drying said liquid, then distributively injecting a liquid by means of a non-contact-type distributive injector.

50. A manufacturing method in accordance with any one of claims 40-49, wherein resin is poured in from a side gate for injection molding of said container and said cover.

51. A manufacturing method in accordance with any one of claims 40-50, comprising distributively injecting a reagent or sample into the well portions or cover, then welding together said cover and said wells so that each well is liquid-tight.

52. A manufacturing method in accordance with any one of claims 40-50, wherein at least one of a reagent or sample is held in the wells of said container, the other is held on the surface of the cover, and said wells and said cover are joined by ultrasonic welding to induce a reaction between the reagent and sample in each well.

53. An ultrasonic welding apparatus for use in manufacturing microwell arrays, comprising a pressurizing mechanism capable of applying a force of 7000-23000 N during oscillation, and having a maximum oscillation output of 4.1-5.0 kW.

54. An ultrasonic welding apparatus in accordance with claim 53, wherein the horn amplitude is 30-40 microns.

55. An ultrasonic welding apparatus in accordance with claim 54, capable of irradiating with ultrasonic waves in a welding time of within 0.05-0.8 seconds.

56. An ultrasonic welding apparatus in accordance with any one of claims 53-55, capable of welding each well within a time of 0.05-0.8 seconds.

57. A genetic analysis method comprising steps of distributively injecting a reagent or sample into well portions or cover surfaces of a microwell array comprising a container having a plurality of isolated wells arranged in an array, and a cover capable of covering the container, then welding together said cover and said wells so that each well is liquid-tight, and performing fluorescent light intensity analysis for each well after enabling the reagent and sample to react, or while enabling the reagent and sample to react, thereby to analyze the degree of reaction or the genes in each well.

58. An analysis method in accordance with claim 57, for analyzing genetic polymorphism.

59. An analysis method in accordance with claim 57 or 58, comprising appending a bar code corresponding to each reagent and sample distributively injected into the microwell array, and enabling the progress to be managed by the bar code for each step or each microwell array.

60. An analysis method in accordance with any one of claims 57-59, comprising distributively injecting different DNA into each well, next distributively injecting reagent into the plurality of said wells, then welding together said wells and said cover, enabling the reagent to react with the DNA, and analyzing the fluorescent light intensity of each well to perform polymorphic typing.

61. An analysis method in accordance with any one of claims 57-59, comprising distributively injecting a reagent into each well, next distributively injecting different DNA into the plurality of said wells, then welding together said wells and said cover, enabling the reagent to react with the DNA, and analyzing the fluorescent light intensity of each well to perform polymorphic typing.

62. An analysis method in accordance with any one of claims 57-59, comprising distributively injecting a reagent onto the cover surface, next distributively injecting different DNA into the plurality of said wells, then welding together said wells and said cover, enabling the reagent to react with the DNA, and analyzing the fluorescent light intensity of each well to perform polymorphic typing.

63. A genetic diagnosis method comprising steps of distributively injecting different reagents onto either one of a cover surface or into the wells of a microwell array comprising a container having a plurality of isolated wells arranged in an array, and a cover capable of covering the container, next distributively injecting different DNA into the plurality of said wells, then welding together said wells and said cover, enabling the reagent to react with the DNA, and analyzing the fluorescent light intensity of each well to perform genetic polymorphism analysis.

## Fig. 1

Liquid Expelling Portion
Cover
Through Hole
A
A'
Fitting Portion (Inserting Portion)
Guide Pin
Fitting Portion (Receiving Portion)
Container
Well

## Fig. 2

A — A'

Liquid Expelling
Cover
Raised Portion
Liquid Runoff Channel
Container
Well Mouth Portion

**Fig. 3**

(A)

(B)

(C)

(D)

(E)

(F)

(G)

(H)

**Fig. 4**

Cover

Inserting Portion

Receiving Portion

Container

**Fig. 5**

Well

Liquid Runoff
Channels
(Hatched Portions)

Fig. 6

Well

Liquid Runoff
Channels
(Hatched Portions)

Fig. 7

Excitation
Beam

Excitation
Beam

Fluorescent
Light

Fluorescent
Light

Light-Reflecting
Object

**Fig. 8**

Skirt Portion

Side Gate

Through Hole

Side Gate

Skirt Portion

**Fig. 9**

(A) Spotting of Genomic DNA

(B) Spotting of Reagent

(C) Placement of Cover

(D) Pressing of Cover

(E) Ultrasonic Welding

**Fig. 10**

(A) Spotting of Genomic DNA

(B) Spotting of Reagent

(C) Placement of Cover

(D) Pressing of Cover

(E) Ultrasonic Welding

**Fig. 11**

Contact-Type
Spotting

Non-contact
Type
Spotting

Seal

**Fig. 12**

Quantity of Fluorescent Light Detected After Reaction (%) vs. Ultrasonic Welding Time (sec)

**Fig. 13**

Quantity of Liquid Sealed in Wells (%) vs. Pressure Applied Immediately Before Vibration (N/cm)

**Fig. 14**

(a) Typing Method of Present Invention

384 Types DNA in Container → Reagent in Container → Weld

(b) Typing Method of Present Invention

1 Type Reagent in Container → 384 Types DNA in Container → Weld

(c) Typing Method of Present Invention

1 Type Reagent on Cover → 384 Types DNA in Container → Weld

(d) Genetic Diagnois of Present Invention

384 Types Reagent on Cover → 1 Type DNA in Container → Weld

(e) Genetic Diagnosis of Present Invention

384 Types Reagent in Container → 1 Type DNA in Container → Weld

(f) Conventional Typing Method Using Microtiter Plate

1 Type Reagent → 384 Types DNA → Seal with Adhesive Film

**Fig. 15**

Cover

Through Hole

B

B'

Fitting Portion
(Inserting Portion)

Intermediary Body

Guide Pin

Fitting Portion
(Receiving Portion)

Container

Well

**Fig. 16**

B —————————————————————————————————— B'

Cover

Intermediary Body

Raised Portion

Liquid Runoff
Channel

Container

Well Mouth Portion

**Fig. 17**

(A) Spotting of Genomic DNA

⇩

(B) Spotting of Reagent

⇩

(C) Placement of Intermediary Member

⇩

(D) Pressing of Cover

⇩

(E) Joining by Fitting

**Fig. 18**

Well

Recessed Portion for Adhesive or Raised Portion for Ultrasonic Welding

**Fig. 19**

Well

Recessed Portion for Adhesive or Raised Portion for Ultrasonic Welding

**Fig. 20**

Well

Fitting Portion and
Raised portions for
Ultrasonic Welding

**Fig. 21**

(A) Spotting of Genomic DNA

(B) Spotting of Reagent

(C) Placement of Intermediary Member

(D) Pressing of Cover

(E) Joining by Fitting

**Fig. 22**

(A) Spotting of Genomic DNA

(B) Spotting of Reagent

(C) Placement of Intermediary Member

(D) Pressing of Cover

(E) Joining by Fitting

**Fig. 23**

(A) Spotting of Genomic DNA

(B) Spotting of Reagent

(C) Placement of Intermediary Member

(D) Pressing of Cover

(E) Joining by Fitting

**Fig. 24**

(A) Spotting of Genomic DNA

(B) Spotting of Reagent

(C) Placement of Cover

(D) Pressing of Cover

(E) Ultrasonic Welding

**Fig. 25**

**Fig. 26**

**Fig. 27**

**Fig. 28**

**Fig. 29**

**Fig. 30**

C

C

**Fig. 31**

**Fig. 32**

**Fig. 33**

**Fig. 34**

**Fig. 35**

**Fig. 36**

**Fig. 37**

**Fig. 38**

**Fig. 39**

## INTERNATIONAL SEARCH REPORT

| | International application No. |
| --- | --- |
| | PCT/JP01/08079 |

**A. CLASSIFICATION OF SUBJECT MATTER**
Int.Cl$^7$   G01N35/00, G01N37/00, G01N33/48, C12M1/00, C12Q1/68

According to International Patent Classification (IPC) or to both national classification and IPC

**B.   FIELDS SEARCHED**

Minimum documentation searched (classification system followed by classification symbols)
Int.Cl$^7$   G01N35/00-35/10, G01N37/00, G01N33/48-33/98, C12M1/00-1/42,
C12Q1/00-1/68

Documentation searched other than minimum documentation to the extent that such documents are included in the fields searched
Jitsuyo Shinan Koho        1922-1996    Toroku Jitsuyo Shinan Koho  1994-2001
Kokai Jitsuyo Shinan Koho   1971-2001    Jitsuyo Shinan Toroku Koho  1996-2001

Electronic data base consulted during the international search (name of data base and, where practicable, search terms used)
JICST FILE (JOIS)
WPI/L (QUESTEL)

**C.  DOCUMENTS CONSIDERED TO BE RELEVANT**

| Category* | Citation of document, with indication, where appropriate, of the relevant passages | Relevant to claim No. |
| --- | --- | --- |
| A | WO 96/39481 A (Chiron Corporation),<br>12 December, 1996 (12.12.96),<br>Full text; Figs. 1 to 10<br>& US 5604130 A      & EP 828560 A<br>& JP 11-507508 A | 1-65 |
| A | US 5487872 A (Molecular Device Corporation),<br>30 January, 1996 (30.01.96),<br>Full text; Figs. 1 to 7   (Family: none) | 1-65 |
| A | JP 9-99932 A (Dainippon Printing Co., Ltd.),<br>15 April, 1997 (15.04.97),<br>Full text; Figs. 1 to 14   (Family: none) | 1-65 |
| A | JP 62-195849 A (Japan Storage Battery Co., Ltd.),<br>28 August, 1987 (28.08.87),<br>Full text; Figs. 1 to 2   (Family: none) | 1-65 |

☐  Further documents are listed in the continuation of Box C.     ☐  See patent family annex.

| | |
| --- | --- |
| *    Special categories of cited documents:<br>"A"   document defining the general state of the art which is not considered to be of particular relevance<br>"E"   earlier document but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search<br>10 December, 2001 (10.12.01) | Date of mailing of the international search report<br>18 December, 2001 (18.12.01) |
| --- | --- |
| Name and mailing address of the ISA/<br>Japanese Patent Office | Authorized officer |
| Facsimile No. | Telephone No. |

Form PCT/ISA/210 (second sheet) (July 1992)